# FASCICULE DE BREVET EUROPEEN

(11) **EP 1 348 695 B1**
(45) Date de publication et mention de la délivrance du brevet: **25.04.2012**
(21) Numéro de dépôt: 03290597.8
(22) Date de dépôt: 11.03.2003
(51) Int. Cl.: C07D 207/14, C07D 521/00, C07F 9/09, C07D 417/04, C07D 401/04, C07D 487/08, A61Q 5/10, A61K 8/49

(54) **Dérivés de paraphénylènediamine à groupement pyrrolidinyle substitués par un radical cationique et utilisation de ces dérivés pour la coloration de fibres kératiniques**
Paraphenylendiaminderivate, die durch kationische Radikale substituierte Pyrrolidine enthalten, sowie deren Verwendung für die Färbung von Keratinfasern
Derivatives of paraphenylene diamine with pyrrolidine groups substituted by cationic radicals and their use for dyeing keratinic fibre

(30) Priorité: 27.03.2002 FR 0203847
(43) Date de publication de la demande: 01.10.2003
(73) Titulaire: L'Oréal, 75008 Paris (FR)
(72) Inventeur: Sabelle, Stéphane, 75005 Paris (FR); Ramos, Laure, 92340 Bourg Lareine (FR); Leduc, Madeleine, Rés. Les Chèvrefeuilles, 75011 Paris (FR); Philippe, Michel, 91320 Wissous (FR)
(74) Mandataire: Fevrier, Murielle Françoise E.

(56) Documents cités:
- EP-A- 1 018 508
- WO-A-01/68043
- WO-A-99/03819
- FR-A- 2 766 178
- US-A- 5 135 543

## Description

L'invention a pour objet de nouveaux dérivés de paraphénylènediamine à groupement pyrrolidinyle substitués par un radical cationique, les compositions tinctoriales les contenant ainsi que le procédé de teinture de fibres kératiniques à partir de ces compositions.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine, la paratoluène diamine sont des bases d'oxydation largement utilisées. Elles permettent d'obtenir avec des coupleurs d'oxydation des nuances variées.

Cependant, ii existe un besoin de découvrir de nouvelles base d'oxydation présentant un meilleur profil toxicologique que la para-phénylènediamine et la paratoluène diamine, tout en permettant de conférer aux cheveux d'excellente propriétés d'intensité de couleur, de variété de nuances, d'uniformité de la couleur et de ténacité aux agents extérieurs

Il est déjà connu d'utiliser des dérivés de paraphénylènediamine subtitués par un groupement pyrrolidinique comme base d'oxydation pour la coloration de fibres kératiniques. Par exemple, le brevet US 5,851,237 décrit l'utilisation de dérivés 1-(4-aminophényl)pyrrolidine éventuellement substitués sur le noyau benzénique afin de remplacer la paraphénylènediamine.

Le brevet US 5,993,491 propose l'utilisation de dérivés de N-(4-aminophényl)-2-hydroxyméthylpyrrolidine éventuellement substitués sur le noyau benzènique et sur l'hétérocycle pyrrolidinique en position 4 par un radical hydroxy afin de remplacer la para-phénylènediamine.

La demande de brevet JP 11-158048 propose des compositions contenant au moins un composé choisi parmi des dérivés de 4-aminoaniline éventuellement substitués sur le noyau benzénique et dont un des atomes d'azote est compris dans un cycle de 5 à 7 chaînons carbonés.

Le document WO 01/68043 décrit déjà des bases d'oxydation de type paraphénylène diamine substitué sur un des atomes d'azote par un radical pyrrolidinyle. Les documents FR 2766178 et WO 99/03819 décrivent par ailleurs des bases d'oxydation cationique. Il est clairement établi que ces composés ne permettent pas de conférer aux cheveux une coloration de qualité équivalente à celle obtenue avec la para-phénylènediamine ou avec la paratoluènediamine du fait d'un manque d'intensité et d'uniformité de la couleur.

Il existe donc un réel besoin de découvrir de nouvelles bases d'oxydation présentant à la fois un bon profil toxicologique et des propriétés telles que les compositions les contenant permettent de conférer aux cheveux d'excellentes propriétés d'intensité de couleur, de variété de nuances, d'uniformité de la couleur et de ténacité vis à vis des différentes agressions extérieures que peuvent subir les cheveux.

Le but de la présente invention est de développer de nouvelles compositions tinctoriales ne présentant pas les inconvénients des bases d'oxydation de la technique antérieure en fournissant de nouvelles compositions tinctoriales pour la teinture de fibres kératiniques qui ne dégradent pas les fibres kératiniques, tout en étant capables d'engendrer des colorations intenses dans des nuances variées, peu sélectives particulièrement résistantes et présentant un bon profil toxicologique.

Ce but est atteint avec la présente invention qui a pour objet des dérivés de paraphénylènediamine substitués par un groupement pyrrolidinyle de formule (I) et leurs sels d'addition 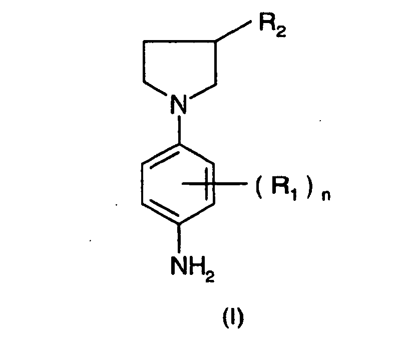 dans laquelle
- n est compris entre 0 et 4, étant entendu que lorsque n est supérieur ou égal à 2 alors les radicaux R₁ peuvent être identiques ou différents,
- R₁ représente un atome d'halogène ; une chaîne hydrocarbonée en C₁-C₆, aliphatique ou alicyclique, saturée ou insaturée, la chaîne pouvant être interrompue par un ou plusieurs atomes d'oxygène, d'azote, de silicium, de soufre ou par un groupement SO₂, ; un radical onium Z, le radical R₁ ne comportant pas de liaison peroxyde, ni de radicaux diazo, nitro ou nitroso ,
- R₂ représente un radical onium Z de formule (II); (III) ou (IV) telle que défini ci après ou un radical -X-C=NR₈-NR₉R₁₀ dans lequel X représente un atome d'oxygène ou un radical -NR₁₁ et R₈, R₉,R₁₀ et R₁₁ représentent un hydrogène, un radical alkyle en C₁-C₄ ou un radical hydroxyalkyle en C₁-C₄

L'invention a aussi pour objet une composition tinctoriale contenant dans un milieu approprié pour la teinture de fibres kératiniques au moins un dérivé de paraphénylènediamine de formule (I) à titre de base d'oxydation.

Un autre objet de l'invention est l'utilisation de cette composition pour la teinture de fibres kératiniques et le procédé de teinture de fibres kératiniques, en particuliers les fibres kératiniques humaines telles que les cheveux mettant en oeuvre de la composition de la présente invention.

La composition de la présente invention permet en particulier d'obtenir une coloration de fibres kératiniques chromatiques, puissante, peu sélective et tenace.

Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne ramifiée saturée ou insaturée ne contenant pas de structure cyclique aromatique.

Lorsque la chaîne est interrompue par un atome Y d'oxygène, de soufre, d'azote, de silicium ou SO₂, on obtient par exemple un motif CH₂-Y-CH₂.

On entend par le terme "onium" un radical quaternaire d'une base azotée.

A titre d'exemple, R₁ peut être un atome de chlore, un radical méthyle, éthyle, isopropyle, vinyle, allyle, méthoxyméthyle, hydroxyéthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1-amino-2-hydroxyéthyle, 1,2-diaminoéthyle, méthoxy, éthoxy, allyloxy, 2-hydroxyéthyloxy.

Dans la formule (I) lorsque n est égal à 1, R₁ est de préférence un atome d'halogène ; une chaîne hydrocarbonée en C₁-C₆, aliphatique ou alicyclique, saturée ou insaturée, un ou plusieurs atomes de carbone pouvant être remplacés par un atome d'oxygène, d'azote, de silicium, de soufre, par un groupement SO₂, le radical R₁ ne comportant pas de liaison peroxyde, ni de radicaux diazo, nitro ou nitroso. De préférence, R₁ est choisi parmi le chlore, le brome, un radical alkyle en C₁-C₄, hydroxyalkyle en C₁-C₄, aminoalkyle en C₁-C₄, alcoxy en C₁-C₄, hydroxyalcoxy en C₁-C₄. A titre d'exemple, R₁ est choisi parmi un radical méthyle, hydroxyméthyle, 2-hydroxyéthyle, 1,2-dihydroxyéthyle, méthoxy, isopropyloxy, 2-hydroxyéthoxy.

Le radical onium Z de formule (II) est 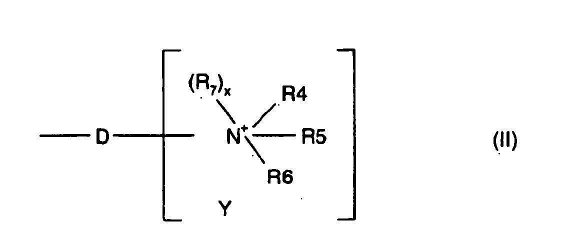 dans laquelle
- D est une liaison covalente ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions cétone ;
- R₄ R₅ et R₆, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆; un radical trialkyl(C₁-C₆)silanealkyle en C₁-C₆ ; un radical aminoalkyle en C₁-C₆ ; un radical aminoalkyle en C₁-C₆ dont l'amine est mono- di-, tri substituée par un radical alkyle en C₁-C₄, alkyl(C₁-C₆)carbonyle, amido ou alkyl(C₁-C₆)sulfonyle; à la condition que lorsque D est une liaison covalente alors R4 représente un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono-di-, tri substituée par un radical alkyle en C₁-C₄, alkyl(C₁-C₆)carbonyle, amido ou alkyl(C₁-C₆)sulfonyle
- R₄, R₅ et R₆ ensemble, deux à deux, forment, avec l'atome d'azote auxquels ils sont rattachés, un cycle saturé carboné à 4, 5, 6 ou 7 chaînons pouvant contenir un ou plusieurs hétéroatomes tel que par exemple un cycle azétidine, un cycle pyrrolidine, un cycle pipéridine, un cycle pipérazine ou un cycle morpholine, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkyl(C₁-C₆)carbonyl, un radical thio (-SH), un radical thioalkyle (-R-SH) en C₁-C₆, un radical alkyl(C₁-C₆)thio, un radical amino, un radical amino mono di ou trisubstitué par un radical alkyl(C₁-C₆), alkyl(C₁-C₆)carbonyle, amido ou alkyl(C₁-C₆)sulfonyle ;
- R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆ ; un radical aminoalkyle en C₁-C₆ dont l'amine est mono- ou di-subsituée par un radical alkyl(C₁-C₆), alkyl(C₁-C₆)carbonyle, amido ou alkyl(C₁-C₆)sulfonyle ; un radical carboxyalkyle en C₁-C₆; un radical carbamylalkyle en C₁-C₆ ; un radical trifluoroalkyle en C₁-C₆ : un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₈; un radical N-alkyl(C₁-C₆)sulfonamidoalkyle en C₁-C₆ ;
- x est 0 ou 1,
   - lorsque x = 0, alors le bras de liaison est rattaché à l'atome d'azote portant les radicaux R₄ à R₆,
   - lorsque x = 1, alors deux des radicaux R₄ à R₆ forment conjointement avec l'atome d'azote auquel ils sont rattachés un cycle saturé à 4, 5, 6 ou 7 chaînons et D est lié à un atome de carbone du cycle saturé ;
- Y est un contre ion.

Dans la formule (II), lorsque x est égal à 0, alors R₅ et R₆ séparément sont choisis de préférence parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical amidoalkyle en C₁-C₆, un radical trialkyl(C₁-C₆)silanealkyle en C₁-C₆ , ou R₄ avec R₅ forment ensemble un cycle azétidine, un cycle pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle mono ou disubstitué par un radical alkyl(C₁-C₆), alkyl(C₁-C₆)carbonyle, amido ou alkyl(C₁-C₆)sulfonyle ; un radical carbamylalkyle en C₁-C₆ ; un radical trialkyl(C₁-C₆)Silanealkyle en C₁-C₈ ; un radical alkyl(C₁-C₆)carboxyalkyle en C₁-C₆ ; un radical alkyl(C₁-C₆)carbonyalkyle en C₁-C₆ ; un radical N-alkyl(C₁-C₆)carbamylalkyle en C₁-C₆.

Lorsque x est égal à 1, alors R₇ est de préférence choisi parmi un radical alkyle en C₁-C₆ ; un radical monohydroxyalkyle en C₁-C₆; un radical polyhydroxyalkyle en C₂-C₆ : un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆ ; un radical trialkyl(C₁-Cₑ)silanealkyle en C₁-C₆ ; un radical alkyl(C₁-C₆)carboxyalkyle en C₁-C₆ ; un radical alkyl(C₁-C₆)carbonylalkyle en C₁-C₆ : un radical N-alkyl(C₁-C₆)carbamylalkyle en C₁-C₆ ; R₄ avec R₅ ensemble forment un cycle azétidine, pyrrolidine, pipéridine, pipérazine, morpholine, R₆ étant choisi dans ce cas parmi un radical alkyle en C₁-C₆ : un radical monohydroxyalkyle en C₁-C₆ ; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆; un radical trialkyl(C₁-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamylalkyle en C₁-C₆.

Dans la formule (II), D est de préférence une liaison covalente ou une chaîne alkylène pouvant être substituée.

Le radical onium Z correspondant à la formule (III) est 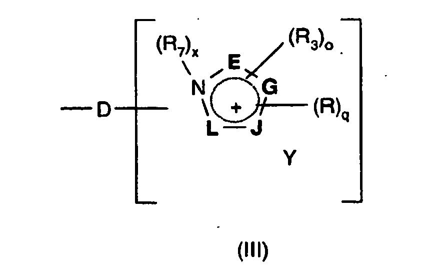 dans laquelle
- D est une liaison covalente ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions cétone;
- les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
- q est un nombre entier compris entre 0 et 4 inclus ;
- o est un nombre entier compris entre 0 et 3 inclus ;
- q+o est un nombre entier compris entre 0 et 4
- R, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thio, un radical thioalkyle en C₁-C₆, un radical alkyl(C₁-C₆)thio, un radical amino, un radical amino mono- ou di-substitué par un radical alkyl(C₁-C₆), alkyl(C₁-C₆)carbonyle, amido, alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
- R₃ , identique ou différent, représente un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₃ sont portés par un azote,
- R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est substitué par un radical alkyl(C_{I}-C₆), alkyl(C₁-C₆)carbonyle, amido ou alkyl(C₁-C₆)sulfonyle ; un radical carboxyalkyle en C₁-C₆; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x est 0 ou 1
   - lorsque x = 0, le bras de liaison D est rattaché à l'atome d'azote,
   - lorsque x = 1, le bras de liaison D est rattaché à l'un des sommets E, G, J ou L,
- Y est un contre-ion.

A titre d'exemple, les sommets E, G, J et L peuvent former un cycle pyrolique, imidazolique, pyrazolique, oxazolique, thiazolique et triazolique, de préférence imidazolique.

Parmi les radicaux R₂ de formules (III), on préfère les radicaux dans lesquels x est égal à 0, D est une liaison covalente ou une chaîne alkylène pouvant être substituée.

Le radical onium Z correspondant à la formule (IV) est 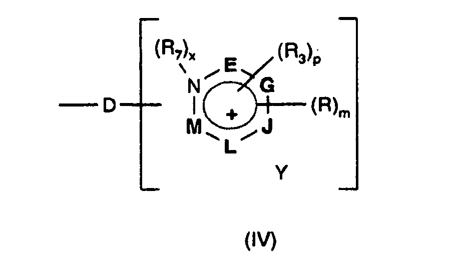 dans laquelle :
- D est une liaison covalente ou une chaîne alkylène en C₁-C₁₄, linéaire ou ramifiée pouvant être interrompue par un ou plusieurs hétéroatomes choisis parmi un atomes d'oxygène, de soufre ou d'azote, et pouvant être substituée par un ou plusieurs radicaux hydroxyle, alcoxy en C₁-C₆ ou amino, et pouvant porter une ou plusieurs fonctions cétone;
- les sommets E, G, J, L et M identiques ou différents, représentent un atome de carbone, d'oxygène, de soufre ou d'azote former un cycle choisi parmi les cycles pyridiniques, pyrimidiniques, pyraziniques, triaziniques et pyridaziniques ;
- p est un nombre entier compris entre 0 et 3 inclus ;
- m est un nombre entier compris entre 0 et 5 inclus ;
- p+m est un nombre entier compris entre 0 et 5 ;
- R, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thio, un radical thioalkyle en C₁-C₆, un radical alkyl(C₁-Cₑ)thio, un radical amino, un radical amino substitué par un radical alkyl(C₁-C₆), alkyl(C₁-C₆)carbonyle, amido ou alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
- R₃, identiques ou différents, représentent un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₃ sont portés par un azote,
- R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C1-C6)silanealkyle en C1-C6; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylaikyle en C₁-C₆ ; un radical alkyl(C₁-C₆)carbonylalkyle en C₁-C₆; un radical N-alkyl(C₁-C₆)carbamylalkyle en C₁-C_{6 ;} un radical N-alkyl(C₁-C₆)sulfonamidoalkyle en C₁-C₆ ;
- x est 0 ou 1
   - lorsque x = 0, le bras de liaison D est rattaché à l'atome d'azote,
   - lorsque x = 1, le bras de liaison D est rattaché à l'un des sommets E, G, J, L ou M,
- Y représente un contre ion.

De préférence, les sommets E,G,J,L et M forment avec l'azote du cycle un cycle pyridinique et pyrimidinique.

Lorsque x est égal à 0 alors R est de préférence choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical alkylcarbonyle en C₁-C₆, un radical amino, un radical amino mono- ou di-substitué par un radical alkyl(C₁-C₆), alkyl(C₁-C₆)carbonyle, amido ou alkyl(C₁-C₆)sulfonyle ; un radical monohydroxyalkyle en C₁-C₆ ou un radical polyhydroxyalkyle en C₂-C₆ et R₃ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbarnylalkyle C₁-C₆.

Lorsque x est égal à 1, R₇ est de préférence choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, un radical amido ou un radical alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carbonylalkyle en C₁-C₆ ; un radical N-alkyl(C₁-C₆)carbamylalkyle en C₁-C₆ ; R est choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical alkylcarbonyle en C₁-C₆, un radical amino, un radical amino mono- ou di- substitué par un radical alkyl(C₁-C₆), alkyl(C₁-C₆)carbonyle, amido ou alkyl(C₁-C₆)sulfonyle; et R₃ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

De préférence, R, R₇ et R₃ sont des radicaux alkyles pouvant être substitués.

Le radical R₂ peut aussi représenter un radical onium de formule -XP(O)(O-)OCH₂CH₂N⁺(CH₃)₃ où X représente un atome d'oxygène ou un radical -NR₁₁, R₁₁ représentant un hydrogène, un radical alkyle en C₁-C₄ ou un radical hydroxyalkyle.

Dans le cadre de l'invention, R₂ peut aussi représenter un radical guanidine de formule -X-C=NR₈-NR₉R₁₀, X représente un atome d'oxygène ou un radical -NR₁₁, R₈, R₉, R₁₀ et R₁₁ représentant un hydrogène, un radical alkyle en C₁-C₄ ou un radical hydroxyalkyle. Selon un mode de réalisation particulier, X est -NR₁₁, R₈ est un hydrogène, R₉ et R₁₀ sont choisis parmi l'hydrogène ou un radical alkyle, de préférence méthyle.

Le pKa du radical guanidine R₂ est en général tel que ce substituant est présent sous forme cationique (=NR₈H⁺) dans les conditions classiques de teinture des cheveux par oxydation.

Dans le cadre de l'invention, le contre ion peut être choisi parmi un atome d'halogène tel que le brome, le chlore, le fluor ou l'iode, un hydroxyde, un citrate, un succinate, un tartrate, un lactate, un tosylate, un mésylate, un benzènesulfonate, un acétate, un hydrogènesulfate ou un alkylsulfate en C₁-C₆ tel que par exemple le méthylsulfate, ou l'éthylsulfate.

A titre d'exemples de dérivés de formule (I), on peut citer :

| Formule | Nomenclature | formule | Nomenclature |
|---|---|---|---|
| 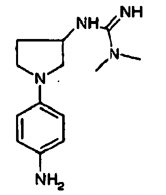 | N'-[1-(4-Amino-phényl)-pyrrolidin-3-yl]-N,N-diméthyl-guanidine | 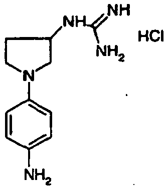 | N-[1-(4-Amino-phényl)-pyrrolidin-3-yl]-guanidine |
| 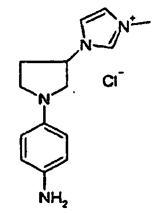 | 3-[1-(4-Amino-phényl)-pyrrolidin-3-yl]-1-méthyl-3H-imidazol-1-ium; chlorure | 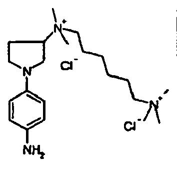 | [1-(4-Amino-phényl)-pyrrolidin-3-yl]-(-triméthylammonium-hexyl)-diméthyl-ammonium; dichlorure |
| 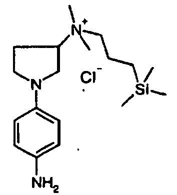 | [1-(4-Amino-phényl)-pyrrolidin-3-yl]-diméthyl-(3-triméthylsilanyl-propyl)-ammonium ; chlorure | 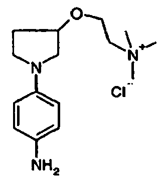 | {2-[1-(4-Amino-phényl)-pyrrolidin-3-yloxy]-ethyl}-triméthyl-ammonium; chlorure |
| 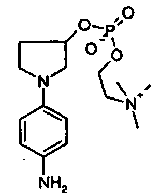 | [1-(4-Amino-phényl)-pyrrolidin-3-yl]-oxophosphorylcholine | 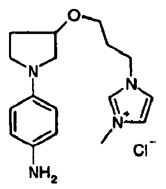 | 3-{3-[1-(4-Amino-phényl)-pyrrolidin-3-yloxy]-propyl}-1-méthyl-3H-imidazol-1-ium ; chlorure |
| 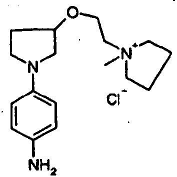 | 1-{2-[1-(4-Amino-phényl)-pyrrolidin-3-yloxy]-ethyl}-1 -méthyl-pyrrolidinium; chlorure | 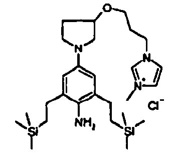 | 3-{3-[1-(5-triméthylsilanylethyl-4-Amino-3- triméthylsilanylethyl -phényl)-pyrrolidin-3-yloxy]-propyl}-1-méthyl-3H-imidazol-1-um ; chlorure |
| 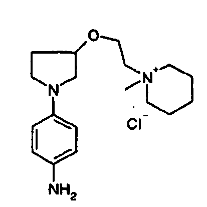 | 1-{2-[1-(4-Amino-phényl)-pyrrolidin-3-yloxy]-ethyl}-1-méthyl-piperidinium; chlorure | 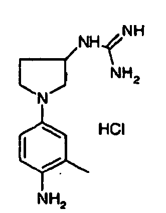 | N-[1-(4-Amino-3-méthyl-phényl)-pyrrolidin-3-yl]-guanidine |
| 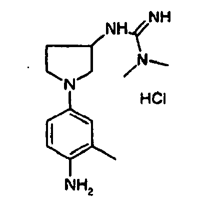 | N'-[1-(4-Amino-3-méthyl-phényl)-pyrrolidin-3-yl]-N,N-diméthyl-guanidine | 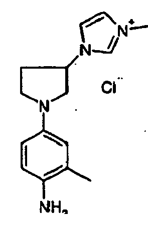 | 3-[1-(4-Amino-3-méthyl-phényl)-pyrrolidin-3-yl]-1-méthyl-3H-imidazol-1-ium; chlorure |
| 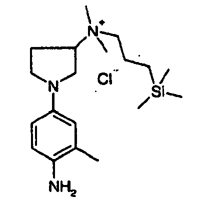 | [1-(4-Amino-3-méthyl-phényl)-pyrrolidin-3-yl]-diméthyl-(3-triméthylsilanyl-propyl ammonium ; chlorure | 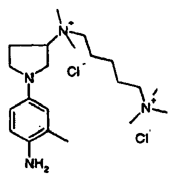 | [1-(4-Amino-3-méthyl-phényl)-pyrrolidin-3-yl]-(-triméthylammonium-hexyl)-diméthyl-ammonium dichlorure |
| 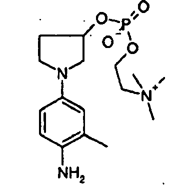 | [1-(4-Amino-3-méthyl-phényl)-pyrrolidin-3-yl]-oxophosphorylcholine | 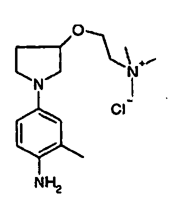 | {2-[1 -(4-Amino-3-méthyl-phényl)-pyrrolidin-3-yloxy]-ethyl}-triméthyl-ammonium; chlorure |
| 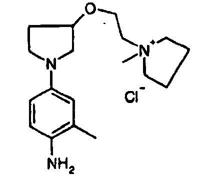 | 1-{2-[1-(4-Amino-3-méthyl-phényl)-pyrrolidin-3-yloxy]-ethyl}-1-méthyl-pyrrolidinium ; chlorure | 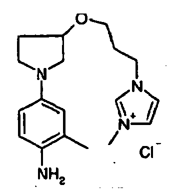 | 3-{3-[1-(4-Amino-3-méthyl-phényl)-pyrrolidin-3-yloxy]-propyl}-1-méthyl-3H-imidazol-1-um ; chlorure |
| 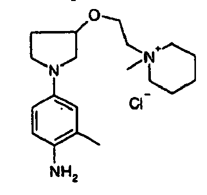 | 1-{2-[1-(4-Amino-3-méthyl-phényl)-pyrrolidin-3-yloxy]-ethyl}-1-méthyl-piperidin | 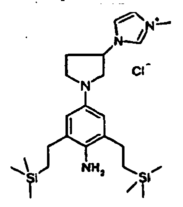 | 3-[1-(5-triméthylsilanylethyl-4-Amino-3-triméthylsilanylethyl-phényl)-pyrrolidin-3-yl]-1-méthyl-3H-imidazol-1-ium; chlorure |
| 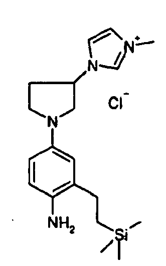 | 3-[1-(4-Amino-3-triméthylsilanylethyl-phényl)-pyrrolidin-3-yl]-1-méthyl-3H-imidazol-1-ium; chlorure | 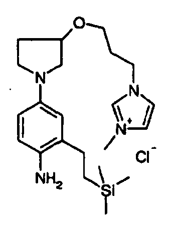 | 3-{3-[1-(4-Amino-3-triméthylsilanylethyl -phényl)-pyrrolidin-3-yloxy]-propyl}-1-méthyl-3H-imidazol-1-um ; chlorure |
| 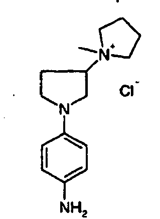 | 1'-(4-Amino-phényl)-1-méthyl-[1,3']bipyrrolidinyl-1 -ium; chlorure | 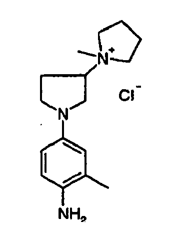 | 1'-(4-Amino-3-méthyl-phényl)-1-méthyl-[1,3']bipyrrolidinyl-1-ium; chlorure |
| 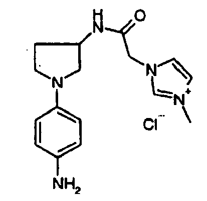 | 3-{[1-(4-Amino-phényl)-pyrrolidin-3-ylcarbamoyl]-méthyl}-1-méthyl-3H-imidazol-1-ium ; chlorure | 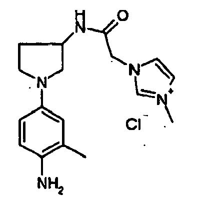 | 3-{[1-(4-Amino-3-méthyl-phényl)-pyrrolidin-3-ylcarbamoyl}-méthyl}-1-méthyl-3H- imidazol-1-ium ; chlorure |
| 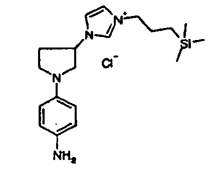 | 3-[1-(4-Amino-phényl)-pyrrolidin-3-yl]-1-(3-triméthylsilanyl-propyl)-3H-imidazol-1-ium ; chlorure | 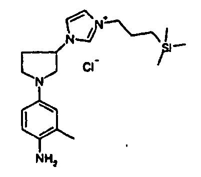 | 3-[1-(4-Amino-3-méthyl-phényl)-pyrrolidin-3-yl]-1-(3-triméthylsilanyl-propyl)-3H-imidazol-1-ium ; chlorure |
| 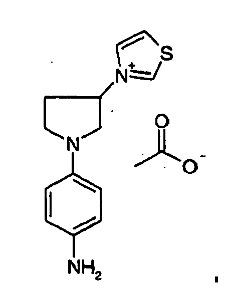 | Acétate de 3-[1-(4-Amino-phenyl)-pyrrolidin-3-yl]-thiazol-3-ium | 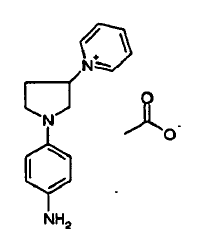 | Acétate de 1-[1-(4-aminophenyl)pyrrolidin-3-yl]pyridinium |
| 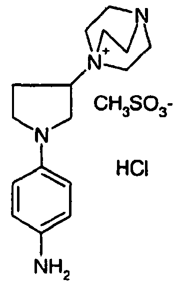 | 1-[1-(4-Amino-phenyl)-pyrrolidin-3-yl]-4-aza-1-azonia-bicyclo[2.2.2] octane ;méthanesulfonate ; chlorhydrate | | |

Parmi ces composés, les composés suivants sont particulièrement préférés :
- N'-[1-(4-Amino-phényl)-pyrrolidin-3-yl]-N,N-diméthyl-guanidine
- N-[1-(4-Amino-phényl)-pyrrolidin-3-yl]-guanidine
- 3-[1-(4-Amino-phényl)-pyrrolidin-3-yl]-1-méthyl-3H-imidazol-1-ium; chlorure
- [1-(4-Amino-phényl)-pyrrolidin-3-yl]-diméthyl-(3-triméthylsilanyl-propyl)-ammonium ; chlorure
- N'-[1-(4-Amino-3-méthyl-phényl)-pyrrolidin-3-yl]-N,N-diméthyl-guanidine
- N-[1-(4-Amino-3-méthyl-phényl)-pyrrolidin-3-yl]-guanidine
- 3-[1-(4-Amino-3-méthyl-phényl)-pyrroiidin-3-yl]-1-méthyl-3H-imidazol-1-ium; chlorure
- [1-(4-amino 3-méthyl phényl) pyrrolidin 3 -yl] diméthyl (3-triméthylsilanyl propyl ammonium ; chlorure ;
- 3-[1-(4-Amino-phényl)-pyrrolidin-3-yl]-1-(3-triméthylsilanyl-propyl)-3H-imidazol-1-ium ; chlorure
- 3-[1-(4-Amino-3-méthyl-phényl)-pyrrolidin-3-yl]-1-(3-triméthylsilanyl-propyl)-3H-imidazol-1-ium ; chlorure
- 1'-(4-Amino-phényl)-1-méthyl-[1,3']bipyrrolidinyl-1-ium; chlorure
- 1'-(4-Amino-3-méthyl-phényl)-1-méthyl-[1,3']bipyrrolidinyl-1-ium; chlorure
- 3-{[1-(4-Amino-phényl)-pyrrolidin-3-ylcarbamoyl]-méthyl}-1-méthyl-3H-imidazol-1-ium ; chlorure 3-{[1-(4-Amino-3-méthyl-phényl)-pyrrolidin-3-ylcarbamoyl]-méthyl}-1-méthyl-3H-imidazol-1-ium ; chlorure
- 1'-(4-amino-phényl)-1-méthyl-[1,3']bipyrrolidinyl-1-ium, chlorure.

Selon un mode de realisation particulier, la chaine D comprend un radical phosphoryl. A titre d'exemple, on peut citer [1-(4-Amino-phenyl)-pyrrolidin-3-yl]-oxophosphorylcholine, [1-(4-Amino-3-méthyl-phenyl)-pyrrolidin-3-yl]-oxophosphorylcholine .

La composition tinctoriale de la présente invention comprend, dans un milieu approprié pour la teinture des fibres kératiniques, en particulier les cheveux humains, à titre de base d'oxydation un dérivé de formule (I) tel que défini précédemment.

La ou les bases d'oxydation de l'invention sont en général présentent chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-R-hydroxyéthylamino-3,4-méthytènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les paraphénylènediamines autres que celles décrites précédemment, les bis-phénylalkylènediamines, les para-aminophénols, les bis-paraaminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la, N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique; la 2-méthoxy-pyrazolo[1,5-a]pyridine-3-ylamino ; le (3-amino-pyrazolo[1,5-a]pyridine-7-yl)-méthanol; le 2-(3-amino-pyrazolo[1,5-a]pyridine-5-yl)-éthanol; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrlmldine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 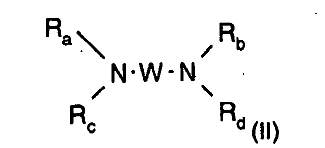 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

La présente invention a aussi pour objet des composés nitro de formule (I') suivante qui sont des composés intermédiaires dans la synthèse des dérivés de formule (I). 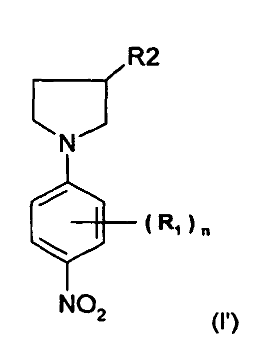 dans laquelle R₁, n et R2 sont tels que définis précédemment.

Les exemples qui suivent servent à illustrer l'invention sans toutefois présenter un caractère limitatif.

« Les composés de la présente invention peuvent être obtenus par application ou adaptation des méthodes connues. En particulier, ils peuvent être obtenus par adaptation des méthodes décrites précédemment. »

### EXEMPLES

### Exemple 1 : synthèse du 1-méthyl-3-[1-(4-amino-phényl)-pyrrolidin-3-yl]-3H-imidazol-1-ium, chlorure : chlorhydrate

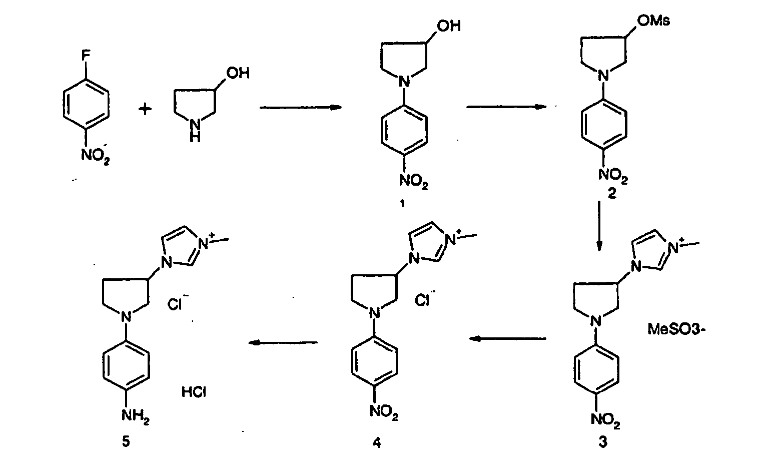

### Synthèse du 1-(4-nitrophényl)-pyrrolidin-3-ol (1)

Dans un tricol, on introduit 2 g de 1-fluoro-4-nitrobenzène (0,0155 mol), 1,3 g d'hydrogénocarbonate de sodium (0,0155 mol) et 15 ml d'un mélange dioxanne/eau (8/2). A ce mélange, on ajoute rapidement 1,35 g de 3-pyrrolidinol (0,0155 mol) racémique. Le mélange hétérogène est chauffé au reflux (87°C) pendant 10 heures. Le mélange réactionnel est ensuite versé dans de l'eau glacée ; on obtient un précipité jaune qui est filtré et rincé à l'eau. Après séchage sous vide en présence de P2O5, 2,95 g d'un solide jaune ont été obtenus (rendement 97%).

RMN ¹H (DMSO d₆, 200 MHz. ppm) conforme au produit attendu : 8,04 (d, J = 9 Hz, 2H) ; 6,58 (d, J = 9 Hz, 2H) ; 5,06 (d, J =3,6 Hz, 1H) ; 4,41 (m, 1H) ; 3,45 (m, 3H) ; 3,20 (m, 1 H) ; 2,04 (m, 2H).:

| Analyse élémentaire : | | | | |
|---|---|---|---|---|
| % | C | H | N | O |
| calculé | 57,89 | 5,81 | 13,45 | 23,05 |
| trouvé | 57,17 | 5,72 | 13,23 | 23,28 |

### Synthèse de l'ester d'acide méthanesulfonique du 1-(4-amino-phényl)-pyrrolidin-3-yl (2)

A 83,3g (0,4mole) de N-(4-nitrophényl)-3-hydroxypyrrolidine (1) en solution dans 625ml de THF anhydre et 72,7ml (0,6mole ) de triéthylamine, on ajoute goutte à goutte 40ml (0,516mole ) de chlorure de mésyle à 5°C. On laisse revenir à température ambiante, puis on verse dans la glace. Après essorage et séchage du précipité, on obtient 109 g de poudre jaune (2).

Point de fusion : 203°C
RMN 1H (400MHz-DMSO) ppm 8.09(d, 2 H) ; 6.68(d, 2H) ;5.47(m, 1H) ; 3.77-3.48 (m,4H) ; 3.28(s,3H) ; 2.35(m,2H).
Masse ESI+ : m/z=287 [MH+]

### Synthèse du 1-méthyl-3-[1-(4-nitro-phényl)-pyrrolidin-3-yl]-3H-imidazol-1-ium ; méthanesulfonate (3)

23g (0,08mole) de l'ester de l'acide méthanesulfonique du 1-(4-nitro-phényl)-pyrrolidin-3yl (2) sont chauffés 8 heures à 85°C dans 150g de 1- méthylimidazole (1,82 moles). Cette solution est agitée dans 2 I d'acétate d'éthyle jusqu'à cristallisation. Après filtration et séchage, on obtient 24g de poudre jaune (3) .

RMN 1H (400MHz-DMSO) ppm 9.44(s, 1H) ; 8.29 (d, 2 H) 8.07(m, 1H) 7.96(m, 1H) ; 6.91(d, 2H) ;5.48(m, 1H) ; 4.16(m, 1H) ; 4.02(s, 3H)de 3.98 à 3.79 à (m,4H) ; 2.85(m,1 H) ; 2.69(m,1 H) ; 2.50(s,3H)
Masse ESI+ : m/z=273 [M+]

### Synthèse du 1-méthyl-3-[1-(4-nitro-phényl)-pyrrolidin-3-yl]-3H-imidazol-1-ium; chlorure (4)

23g (0,0624mole) de 1-méthyl-3-[1-(4-nitro-phényl)-pyrrolidin-3-yl]-3H-imidazol-1-ium ; méthane sulfonate (3) en solution dans 200 ml d'eau sont agités avec 500g de résines échangeuses d'ions Amberlite IRA-402 pendant 14 heures ; la résine est séparée par filtration, le filtrat est concentré puis repris dans de l'isopropanol. La poudre jaune est récupérée par filtration puis séchée. On obtient 16,5g de 1-méthyl-3-[1-(4-nitro-phényl)-pyrrolidin-3-yl]-3H-imidazol-1-ium ; chlorure (4).

RMN 1H (400MHz-DMSO) ppm 9.35(s, 1H) ; 8.11 (m, 2 H) ; 8.90(m, 1H) ; 7.78(m, 1H) ; 6.71(m, 2H) ; 5.31(m, 1H) ; 4.37(m,1H) ; de 3.98 à 3.95(m, 1H) ; 3.85(s, 3H) ; de 3.83 à 3.69 (m,4H); de 2.51 à 2.49.(m.2H) ;
Masse ESI+ : m/z=307 [M+]

### Synthèse du 1-méthyl-3-[1-(4-amino-phényl)-pyrrolidin-3-yl]-3H-imidazol-1-ium: chlorure, chlorhydrate (5)

16g (0,0518 mole) du dérivé (4) précédent en solution dans 600ml d'éthanol sont hydrogénés en présence de palladium sur charbon sous une pression d'hydrogène de 8 bars. Après filtration du catalyseur, le dérivé (5) attendu est isolé sous forme de chlorhydrate.

RMN 1H (400MHz-DMSO) ppm 8.86(s, 1H); 7.59(t, 1H) ; 7.54(t, 1H) ; 7.41 (m, 2H) ; 6.89(m, 2H) ; 5.33(m,1H) ; 3.97(m, 3H) ; 3.85 (m, 2H) ; 3.71 (m, 1H) ; 3.50 (m, H) ; 2.76(m, 1H) ; 2.51.(m, 1H) ;
Masse ESI+ : m/z=243 [M+]

### Exemple 2 : synthèse du N-[1-(4-Amino-phényl)-pyrrolidin-3-yl]-quaninidine trichlorhydrate

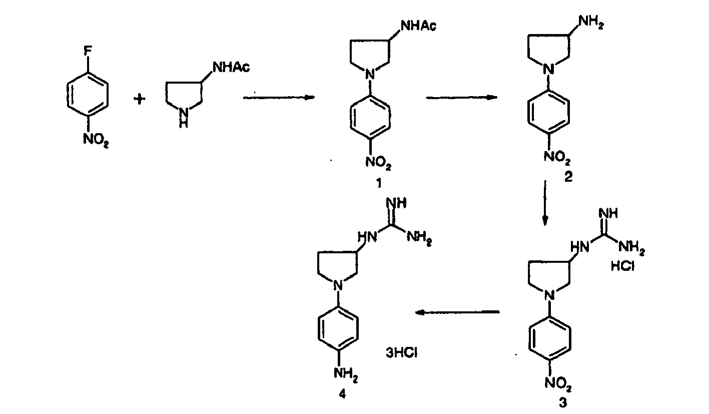

### synthèse du N-[1-(4-Nitro-phényl)-pyrrolidin-3-yl]-acetamide (1)

Après dissolution de 56,4 g de 1-fluoro-4-nitrobenzene (0,4 mole) et de 51,2 g de 3 acétamido pyrrolidine (0,4 mole) dans 400 ml de NMP, on ajoute sous atmosphère d'azote 66,4 g de carbonate de potassium (0,48 mole) et on chauffe à 100°C pendant 18h. On laisse refroidir, puis on coule le milieu réactionnel dans 2 I d'eau. Le précipité jaune formé est filtré, lavé à l'eau puis séché dans une étuve à vide sur P₂O₅. On obtient ainsi 100 g (100%) de N-[1-(4-Nitro-phényl)-pyrrolidin-3-yl]-acetamide (1) sous forme d'un solide jaune.

### synthèse du 1-(4-Nitro-phényl)-pyrrolidin-3-ylamine (2)

Dans un tricol de 2 I, on introduit en suspension 100 g (0,4 mole) de N-[1-(4-Nitro-phényl)-pyrrolidin-3-yl]-acetamide (1) dans une solution contenant 300 ml d'acide chlorhydrique à 37% et 660 ml d'eau. Le milieu réactionnel est chauffé à 90°C pendant 7h45. Après refroidissement, le milieu est neutralisé doucement avec 300 ml de soude aqueuse à 35% (pH=8 environ). Le solide résultant est ensuite filtré puis lavé à l'eau jusqu'à la neutralité des eaux de lavage. Le produit est ensuite séché sous vide sur P₂O₅. On obtient ainsi 74 g (89%) de 1-(4-Nitro-phényl)-pyrrolidin-3-ylamine (2) sous forme d'un solide jaune.

### Synthèse du N-[1-(4-Nitro-phényl)-pyrrolidin-3-yl]-guaninidine (3)

2,07g (0,01 mole) de [1-(4-nitro-phényl)-pyrrolidin-3-yl]-amine (2) sont chauffés à 90°C dans 10 ml de DMF. 1,32g (0,009 mole) de 1-amidinopyrazole monochlorhydrate sont ajoutés lentement au milieu réactionnel. On chauffe 8 heures. On précipite ainsi un solide jaune. Le solide ainsi obtenu est filtré, lavé à l'éthanol et séché sous vide pour obtenir 2g d'une poudre jaune (3) (70%).

RMN 1H (400MHz, DMSO) ppm 8.08 (m, 2H), 6.66 (m, 2H), 4.34 (m, 1H), 3.72 (m, 2H), 3.56 (m,1H), 3.48 (m,1H), 3.33 (m, 1H), 2.31 (m, 1H), 2.02 (m, 1H).
Masse ESI+ : m/z=250(MH+)

### Synthèse du N-[1-(4-Amino-phényl)pyrrolidin-3-yl]-guaninidine : trichlorhydrate (4)

1,8g (0,063 mole) du dérivé précédent (3) en solution dans 50ml l'éthanol et 550 ml d'eau est hydrogéné en présence de palladium sur charbon sous une pression d'hydrogène de 8 bars ; après filtration du catalyseur, le dérivé attendu (4) est isolé sous forme de chlorhydrate (60%).

RMN 1H (400MHz, DMSO) ppm 7.52 (m, 2H), 7.31 (m, 2H), 4.53 (m, 1H), 3.94 (dd, 1H), 3.84 (m, 1H), 3.72 (m, 2H), 2.64 (m, 1H), 2.28 (m, 1H).
Masse ESI+ : m/z=220(MH+)

### Exemple 3: synthèse du chlorure de 1'-(4-Amino-phényl)-1-méthyl-[1,3]bipyrrolidinyl-1-ium ; chlorhydrate

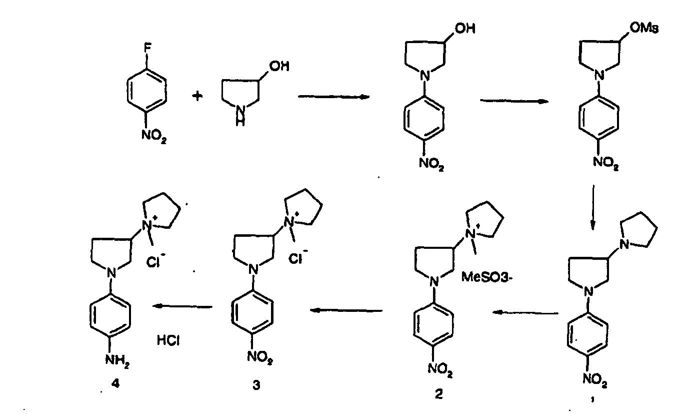

### Synthèse du 1'-(4-Nitro-phenyl)-[1,3']bipyrrolidinyl (1)

5 g (0.0174 mole) de methanesulfonic acid 1-(4-nitro-phenyl)-pyrrolidin-3yl ester 2 sont chauffés 2 heures à 85°C dans 30 ml de pyrrolidine. Ce mélange est versé dans de l'eau glacée jusqu'à cristallisation. Après filtration et séchage on chromatographie la poudre jaune obtenue, éluant dichlorométhane/ méthanol (98/2) et récupère 2.6 g de dérivé (1)
.(rendement 53 %)
point de fusion=114°C

RMN 1H (400MHz, DMSO) ppm 8.04(m, 2H) ; 6.61(m, 2 H) ; 3.60(m, 2H) ; 3.40(m, 1H) ; 3.24(m,1H); 2.86(m,1H) ; 2.50(m, 2H) ; 2.16(m.1H) ; 1.92(m,1H) ; 1.70(m, 4H).
Masse ESI+ : m/z=262( MH+)

### Synthèse du 1-Methyl-1'-(4-nitro-phenyl)-[1,3']bipyrrolidinyl-1-ium; chlorure (3)

24.8g (0,095 mole) de 1'-(4-Nitro-phenyl)-[1,3']bipyrrolidinyl (1) sont agités dans 330 ml d'acétate d'éthyle. 10 ml (0,105mole) de diméthylsulfate sont ajoutés et le mélange réactionnel est chauffé à reflux 4h. On laisse revenir à température ambiante. Le solide jaune est filtré, lavé à l'acétate d'éthyle et séché sous vide. On procède à un échange d'ion sur Amberlite IRA-402. On obtient 21g d'une poudre jaune (3) (75%).

RMN 1H (400MHz, D2O) ppm 7.96(m, 2H) ; 6.51 (m, 2 H) ; 4.3(m, 1H) ; 3.78-3.39(m, 8H); 2.98(s,3H) ; 2.97-2.42(m, 2H) ;2.16(m, 4H)
Masse ESI+ : m/z=276(M+)

### Synthèse du chlorure de 1'-(4-Amino-phényl)-1-méthyl-[1,3']bipyrrolidinyl-1ium; chlorhydrate (4)

21g (0,067 mole) du dérivé précédent (3) en solution dans 700ml d'éthanol est hydrogéné en présence de palladium sur charbon sous une pression d'hydrogène de 10 bars ; après filtration du catalyseur, le dérivé attendu (4) est isolé sous forme de chlorhydrate.

RMN 1H (400MHz, DMSO) ppm 2,29 (m, 4 H) ; 2,49 (m, 1H) ; 2,63 (m, 1H) ; 3,1 (s, 3H) ; 3,35 (m, 1H) ; 3,69 (m, 7H); 3,8 (dd, 1H) ; 4,42 (m, 1H) ; 6,86 (m, 2H) ; 7,35 (m, 2H)
Masse ESI+ : m/z=246(M+)

### Exemple 4 : synthèse du chlorure de 3-[1-(4-Amino-3-methyl-phenyl)-pyrrolidin-3-yl]-1-methyl-3Himidazol-1-ium: chlorhydrate

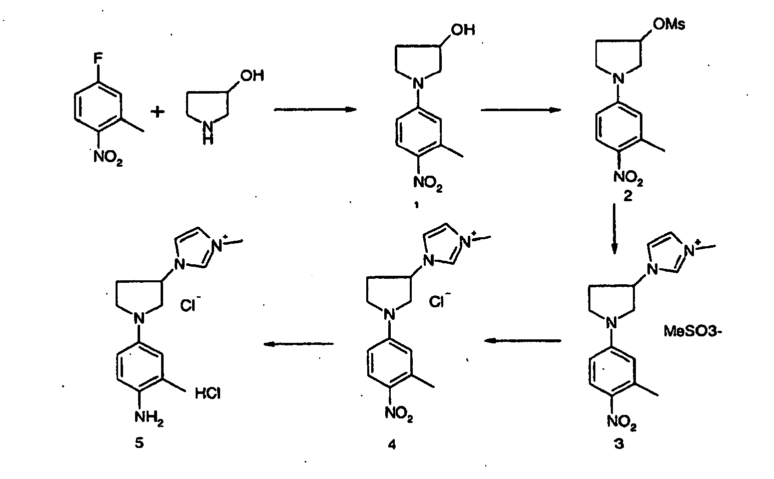

### Synthèse du 1-(3-Methyl-4-nitro-phenyl)-pyrrolidin-3-ol (1)

Dans un tricol, on introduit 38.78g de 5-fluoro-2-nitrotoluène (0,25 mol), 41.4 g de carbonate de potassium (0,3 mol) et 200 ml de N-méthylpyrrolidinone. A ce mélange, on ajoute 26.13 g de 3-pyrrolidinol (0,3 mol). Le mélange hétérogène est agité à température ambiante pendant 12 heures. Le mélange réactionnel est ensuite versé dans de l'eau glacée ; on obtient un précipité jaune qui est filtré et rincé à l'eau. Après séchage sous vide en présence de P2O5, 55.56 g d'un solide jaune ont été obtenus (rendement 95%).

RMN 1H (400MHz-DMSO) ppm 8.01 (d, 1H) ; 6.50-6.46 (m, 2H) ; 5.04(m, 1H) ; 4.42 (m, 1H) 3.50-3.42 (m, 3H) ; 3.24-3.21 (m, 1H) ; 2.56 (s, 3H) ; 2.15-1.90 (m, 2H).

### Synthèse de l'ester d'acide méthane sulfonique du 1-(3-methyl-4-nitro-phenyl)-pyrrolidin-3-yl (2)

A 35.56g (0,16 mole) de 1-(3-Methyl-4-nitro-phenyl)-pyrrolidin-3-ol (1) en solution dans 500ml de THF anhydre et 29ml (0,24mole ) de triéthylamine, on ajoute goutte à goutte 16ml (0,21 mole ) de chlorure de mésyle à 5°C. On laisse revenir à température ambiante, on agite une heure puis on verse dans la glace. Après essorage et séchage du précipité, on obtient 48 g de poudre jaune (2).

RMN 1H (400MHz-DMSO) ppm 7.98-7.95 (m, 1H) ; 6.50-6.47 (m, 2H) ; 5.4 (m, 1H) ; 3.64-3.39 (m, 4H) ; 3.21 (s, 3H) ; 2.50 (s, 3H) ; 2.27-2.24 (m, 2H).

### Synthèse du 1-Methyl-3-[1-(3-methyl-4-nitro-phenyl)-pyrrolidin-3-yl]-3H-imidazol-1-ium ; méthanesulfonate (3)

6g (0,02mole) de l'ester de l'acide méthanesulfonique du 1-(3-methyl-4-nitro-phenyl)-pyrrolidin-3-yl (2) sont chauffés 12 heures à 90°C dans 30ml de 1- méthylimidazole. Cette solution est agitée dans 2 I d'acétate d'éthyle jusqu'à cristallisation. Après filtration et séchage, on obtient 6.6g de poudre jaune (3) .

RMN 1H (400MHz-DMSO) ppm 8.07 (s, 1H) ; 7.72 (m, 1H) ; 7.43 (s, 1H) ; 7.42 (m, 1H) ; 6.28-6.23 (m, 2H) ; 5.16 (m, 1H) ; 3.81-3.77 (m, 4H) ; 3.67-3.43 (m, 3H) ; 2.68 (s, 3H) ; 2.59 (m, 1H) ; 2.35 (m, 1H) ; 2.28 (s, 3H).
Masse ESI+ : m/z= 287[M+]

### Synthèse du 1-Methyl-3-[1-(3-methyl-4-nitro-phenyl)-pyrrolidin-3-yl]-3H-imidazol-1-ium ; chlorure (4)

6.5g (0,017mole) 1-Methyl-3-[1-(3-methyl-4-nitro-phenyl)-pyrrolidin-3-yl]-3H-imidazol-1-ium ; méthanesulfonate (3) en solution dans 200 ml d'eau sont agités avec 200g de résine échangeuse d'ions Amberlite IRA-402 pendant 12 heures; la résine est séparée par filtration, le filtrat est concentré puis repris dans de l'isopropanol. La poudre jaune est récupérée par filtration puis séchée. On obtient 3.4g d'une poudre jaune (4).

RMN 1H (400MHz-DMSO) ppm 8.72 (s,1H) ; 7.65 (m, 1H) ; 7.43 (s, 1H) ; 7.37 (m, 1H) ; 6.22-6.17 (m, 2H) ; 5.16 (m, 1H) ; 3.78 (m, 4H) ; 3.64-3.40 (m, 3H) ; 2.60-2.56 (m, 1H) ; 2.40-2.37 (m, 1H) ; 2.23 (s, 3H).
Masse ESI+ : m/z= 287[M+]

### Synthèse du 1-méthyl-3-[1-(3-methyl-4-amino-phényl)-pyrrolidin-3-yl]-3H-imidazol-1-ium; chlorure, chlorhydrate (5)

3.2g (0,01 mole) du dérivé (4) précédent en solution dans 300ml d'éthanol sont hydrogénés en présence de palladium sur charbon sous une pression d'hydrogène de 9 bars. Après filtration du catalyseur, le dérivé (5) attendu est isolé sous forme de chlorhydrate.

RMN 1H (400MHz-DMSQ) ppm 7,35 (m, 2H) ; 6,86 (m, 2H) ; 4,42 (m, 1H) ; 3,8 (dd , 1H) ; 3,69 (m, 7H) ; 3,35 (m, 1H) ; 3,1 (s, 3H); 2,63 (m, 1H) ; 2,49 (m, 1H) ; 2,29 (m, 4H).Masse ESI+ : m/z= 257[M+]

### Exemple 5 : Acétate de 3-[1-(4-Amino-phenyl)pyrrolidin-3-yl]-thiazol-3-ium

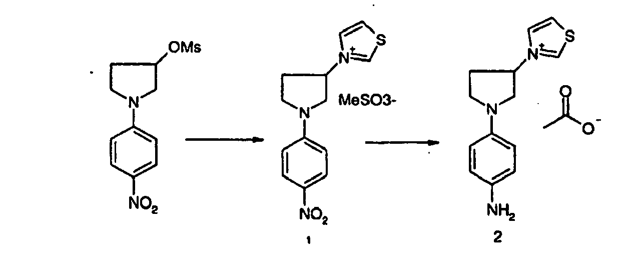

### Synthèse du 3-[1-(4-nitrophenyl)pyrrolidin-3-yl]-1,3-thiazol-3-ium methanesulfonate (1)

Dans un tricol, on introduit 1.14 g de 1-(4-nitrophenyl)pyrrolidin-3-yl methanesulfonate (0,004 mole), 0.01 g de iodure de sodium et 5g (0.059mole) de thiazole. Le mélange hétérogène est chauffé à 110°C pendant 18 heures. Le mélange réactionnel est ensuite versé dans 100ml d'acétate d'éthyle; on obtient un précipité jaune qui est filtré et rincé à l'eau. Après séchage sous vide en présence de P2O5, 1 g d'un solide jaune a été obtenu que l'on recristallise dans l'isopropanol.

RMN 1H (400MHz-DMSO) ppm) 10,26 (dd, 1H) ; 8,64 (dd ,1H) ; 8,41 (dd 1H) ; 8,12 (m ,2 H) ; 6,74 (m ,2 H) ; 5,72 (m, 1H) ; 4,05 (dd ,1H) ; 3,95 (dd , 1H) ; 3,76( m , 1H ) ; 3,62 (m , 1H) ; 2,76( m,1H) ; 2,6 (m, 1H) ; 2,31 (s, 3H).
Masse ESI+ : m/z=270[M+]

### Synthèse de l'acétate de 3-[1-(4-Amino-phenyl)-pyrrolidin-3-yl]-thiazol-3-ium (2)

Après réduction au zinc/ acide acétique on obtient le1-[1-(4-aminophenyl)pyrrolidin-3-yl]thyazolium chloride.
Masse ESI+ : m/z=246[M+]

### Exemple 6 : Acétate de 1-[1-(4-aminophenyl)pyrrolidin-3-yl]pyridinium

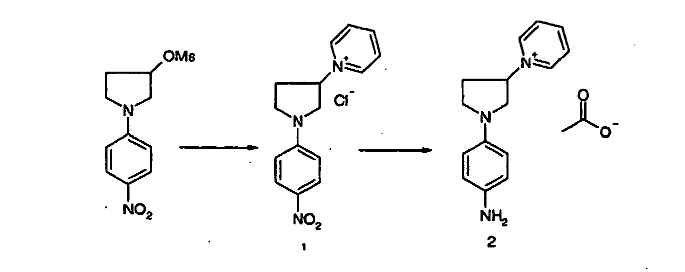

### Synthèse du chlorure de1-[1-(4-Nitro-phenyl)-pyrrolidin-3-yl]-pyridinium (1)

Dans un tricol, on introduit 7g de 1-(4-nitrophenyl)pyrrolidin-3-yl methanesulfonate (0,0244 mole), dans 60 ml de pyridine. Le mélange est chauffé à 100°C pendant 16 heures. On filtre le précipité ; après lavage à l'ether et séchage on obtient 8.5g de poudre jaune. Cette poudre après dissolution dans 200ml d'eau est passée sur résines IRA402 ; après évaporation et séchage on obtient 5.4g de 1-[1-(4-nitrophenyl)pyrrolidin-3-yl]pyridinium chloride. (1)

RMN 1H (400MHz-DMSO) ppm 9,26 (m, 2H) ; 8,67 (m, 1H) ; 8,2 (m, 2H) ; 8,1 (m, 2H) ; 6,74 (m, 1H) ; 5,86 (m, 1H) ; 4,03 (dd, 1H) ; 3,84 (m, 1H) ; 3,62 (m, 1H) ; 2,84 (m, 1H) ; 2,67 (m, 1H).
Masse ESI+ : m/z=270[M+]

### Synthèse de l'acétate de 1-[1-(4-Amino-phenyl)-pyrrolidin-3-yl]-pyridinium (2)

Après réduction au zinc/ acide acétique, on obtient l'acétate de 1-[1-(4-Amino-phenyl)-pyrrolidin-3-yl]-pyridinium.(2)
Masse ESI+ :m/z=240[M+]

### Exemple 7 : 1-[1-(4-Amino-phenyl)-pyrrolidin-3-yl]-4-aza-1-azonia-bicyclo [2.2.2]octane :méthanesulfonate ; chlorhydrate

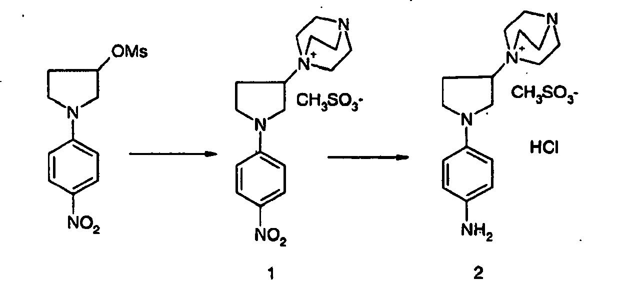

### Synthèse du1-[1-(4-nitrophenyl)pyrrolidin-3-yl]-4-aza-1-azoniabicyclo[2.2.2]octane ; méthanesulfonate

Dans un tricol, on introduit 4,3g de 1-(4-nitrophenyl)pyrrolidin-3-yl methanesulfonate (0,015 mole),15ml de méthylétylcétone et 0,56g (0.005mole) de 1,4 diazabicyclo-2,2,2-octane. Le mélange hétérogène est chauffé à 95°C pendant 10 heures. Le mélange réactionnel est ensuite versé dans 150ml d'eau; l'insoluble est filtré ; la phase aqueuse est extraite au butanol 1 puis est concentrée ; on obtient 0.8g de poudre jaune après séchage correspondant au 1-[1-(4-nitrophenyl)pyrrolidin-3-yl]-4-aza-1-azoniabicyclo[2.2.2]octane ; méthanesulfonate (1)

RMN 1H (400MHz-DMSO) ppm 8,14 (d, 2H) ; 6,72 (d, 2 H) ; 3,67 -4,26 (m, 4H) ; 3,41 (m, 7H) ; 3,06(m, 6H) ; 2,35-2,56 (m, 4H) ; 2,30 (s, 3H).
Masse ESI+ : m/z=303[M+]

### Synthèse du1-[1-(4-Amino-phenyl)-pyrrolidin-3-yl]-4-aza-1-azonia-bicyclo[2.2.2]octane ; méthanesulfonate, chlorhydrate

0.180g de 1-[1-(4-nitrophenyl)pyrrolidin-3-yl]-4-aza-1-azoniabicyclo[2.2.2]octane ; méthanesulfonate sont hydrogénés sous une pression d'hydrogène de 10 bars en présence de palladium sur charbon dans l'éthanol, Après filtration du catalyseur, le dérivé (2) attendu est isolé sous forme de chlorhydrate.

RMN 1H (400MHz-D2O) ppm 7,35 (m, 2H) ; 6,88 (m, 2H) ; 4,6 (m, 1H) ; 4,11 (t, 6H) ; 4,02 (m, 2H) ; 3,96(t, 6H) ; 3,74 (m, 2H) ; 2,8 (s, 3H) ; 2,66 (m, 2H).Masse ESI+ : m/z=373[M+]

### Exemple 8: Dérivés de type [1-(4-amino-phényl)-pyrrolidin-3-yl]-oxophosphorylcholine

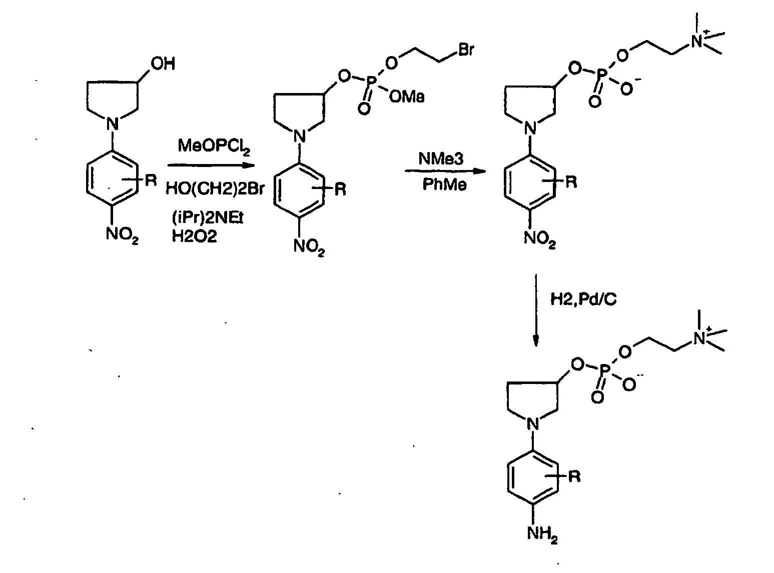

La synthèse des dérivés [1-(4-amino-phényl)-pyrrolidin-3-yl]-oxophosphorylcholine se fait à partir du composé 1-(4-Nitro-phenyl)-pyrrolidin-3-ol suivant le protocole décrit Par S . F. Martin, J. Org. Chem. 1994, 59, 4805-4820.

### Exemples de teinture

### EXEMPLES 1 A 20 DE TEINTURE EN MILIEU ALCALIN

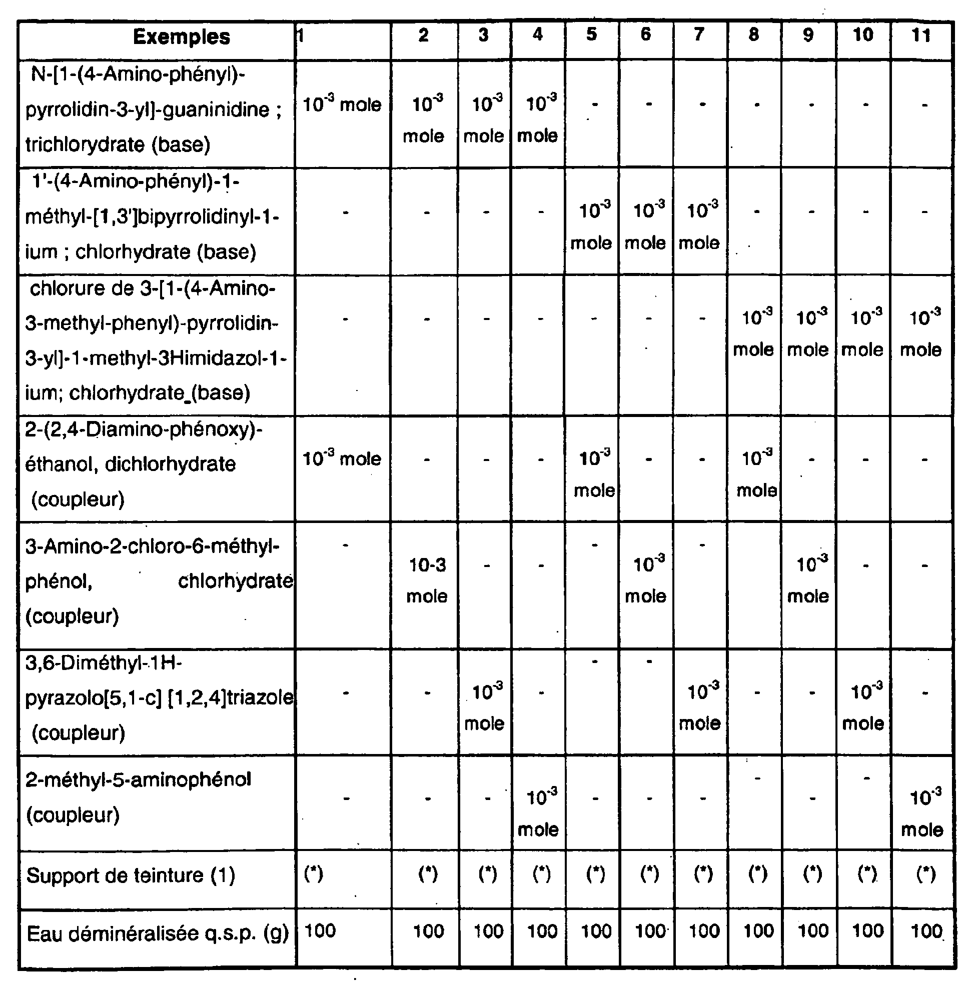

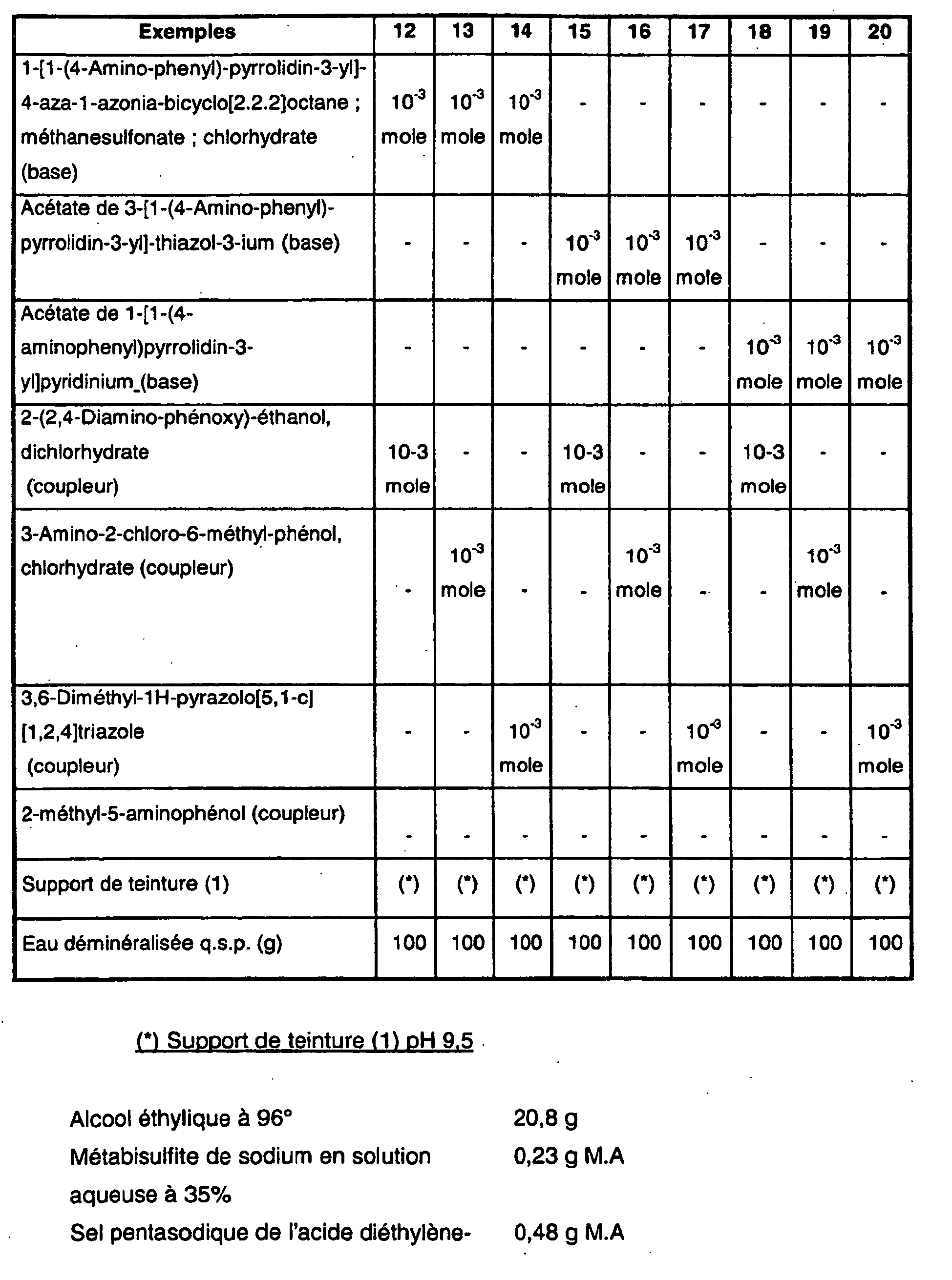

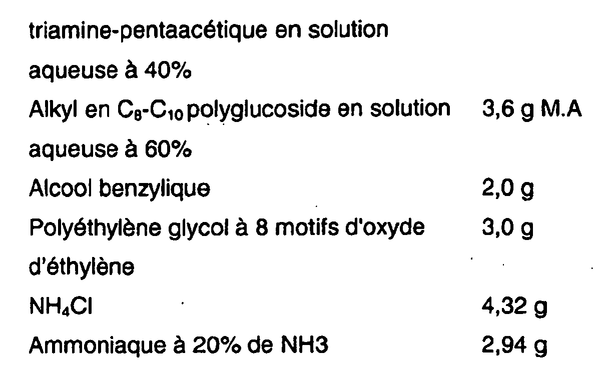

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

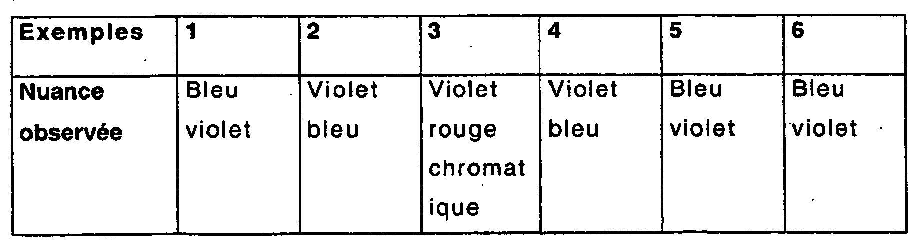

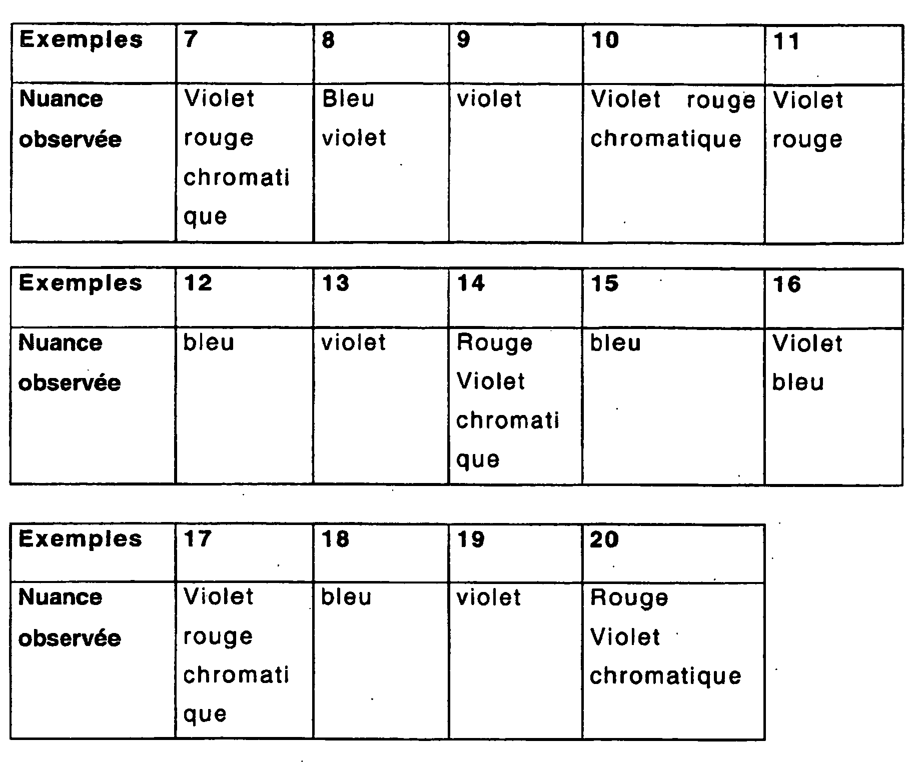

### EXEMPLES 21 A 46 DE TEINTURE EN MILIEU ACIDE

On a préparé les compositions tinctoriales suivantes :

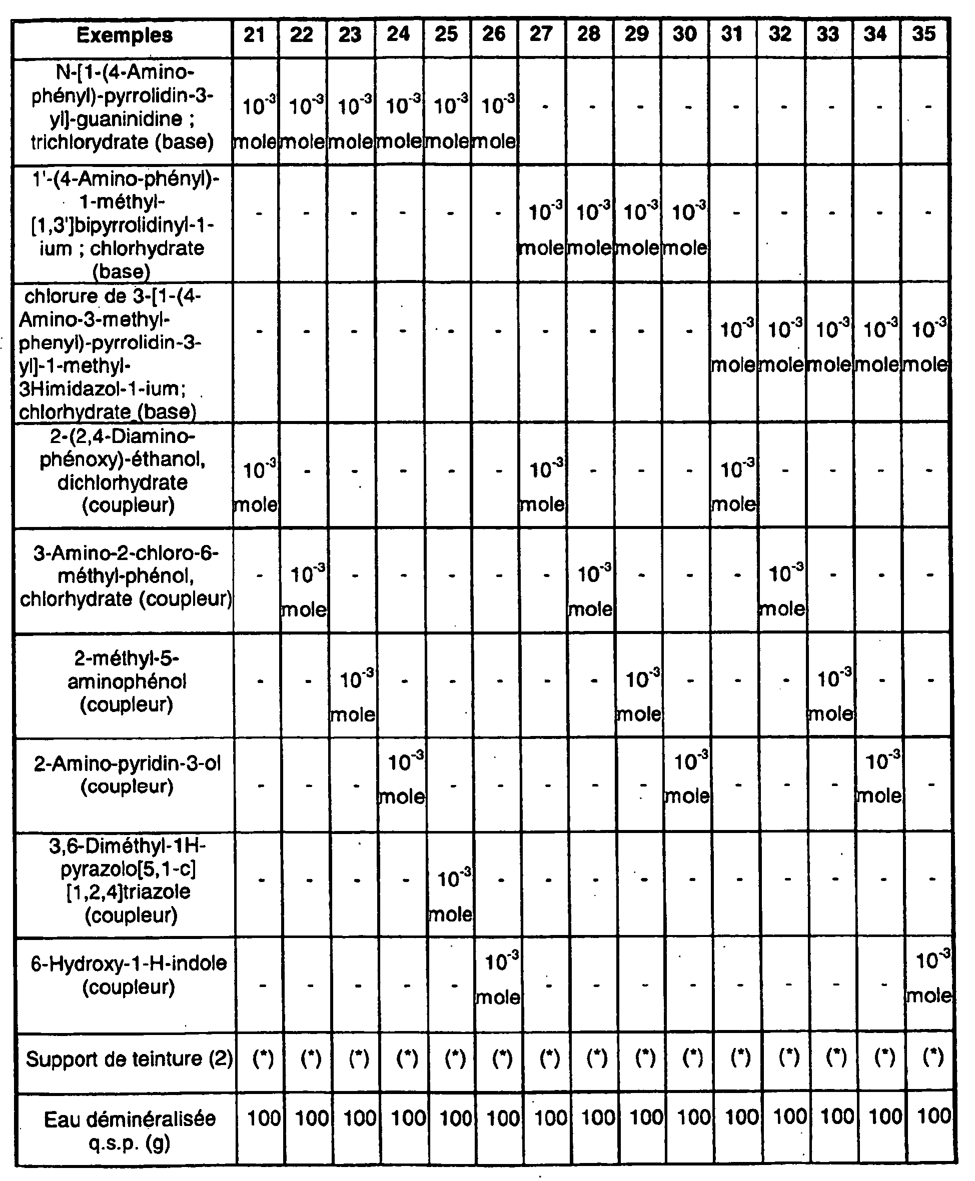

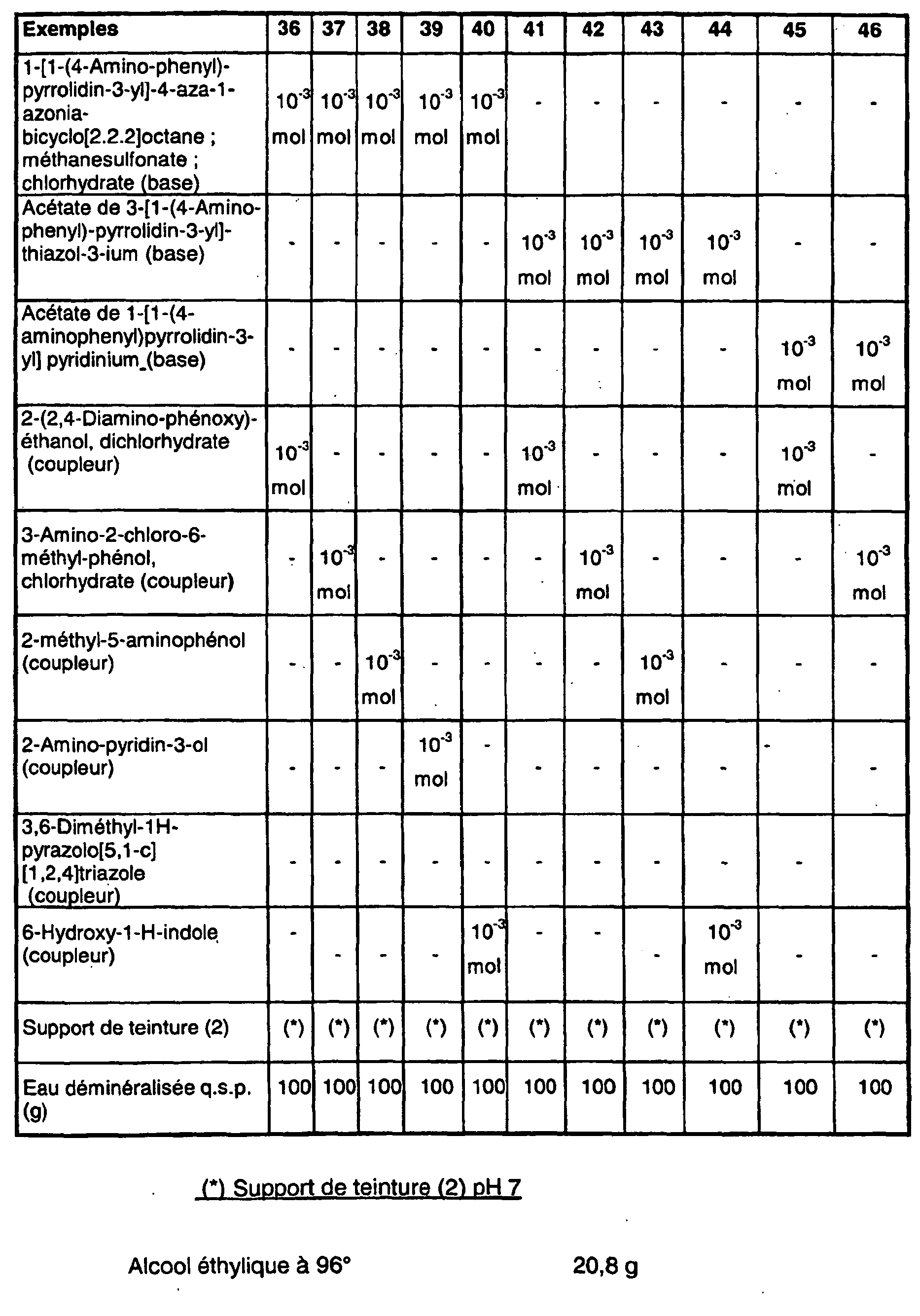

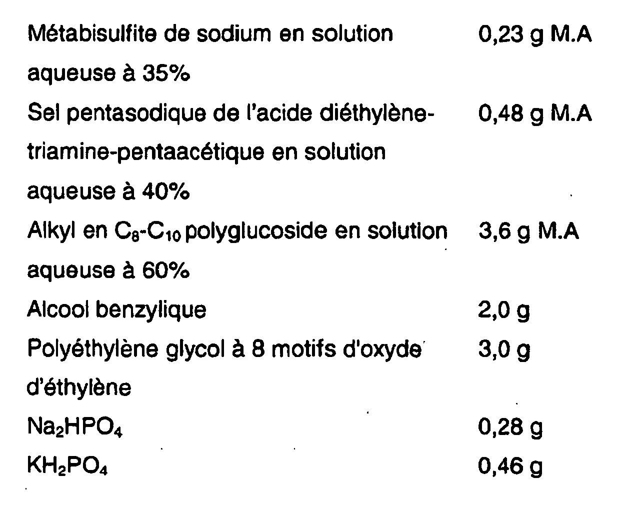

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

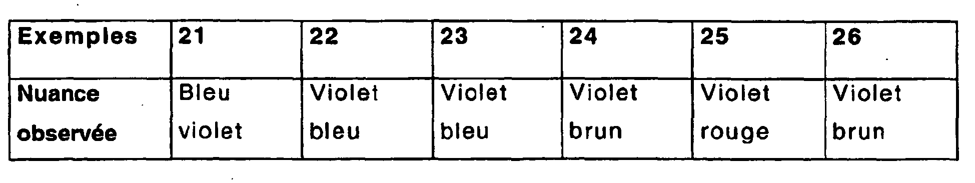

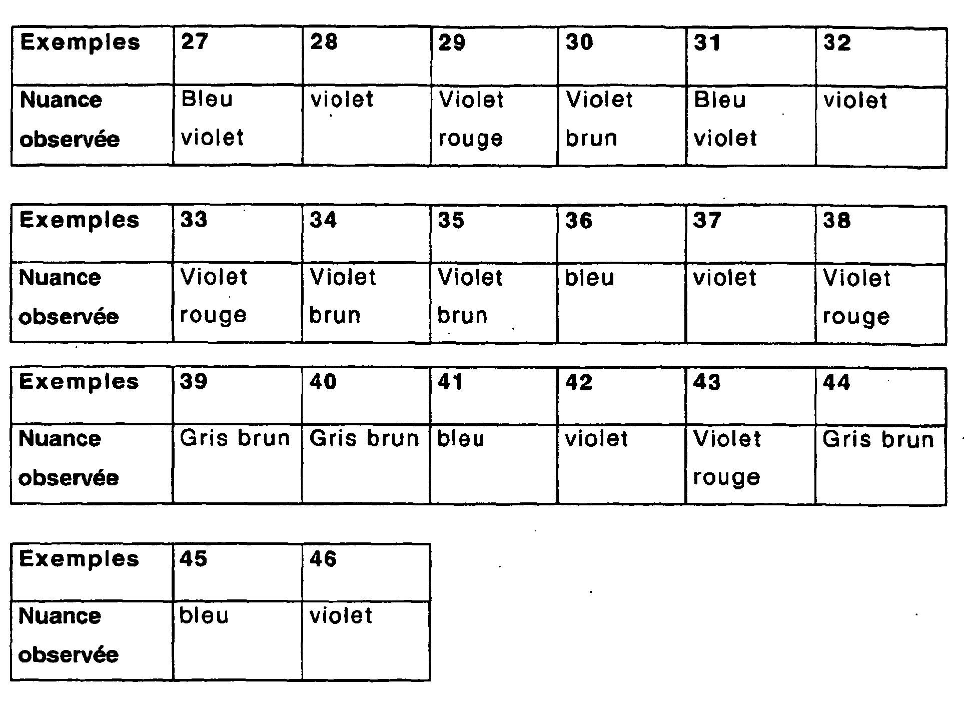

## Revendications

1. Dérivés de paraphénylènediamine substitués par un groupement
pyrrolidinyle de formule (I) et leurs sels d'addition 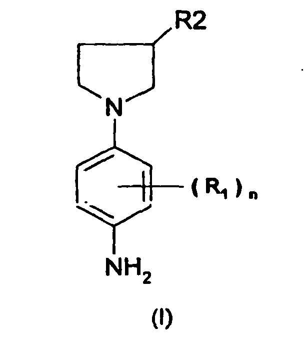 dans laquelle
• n est compris entre 0 et 4, étant entendu que lorsque n est supérieur ou égal à 2 alors les radicaux R₁ peuvent être identiques ou différents,
• R₁ représente un atome d'halogène ; une chaîne hydrocarbonée en C₁-C₈, aliphatique ou alicyclique, saturée ou insaturée, un ou plusieurs atomes de carbone pouvant être remplacés par un atome d'oxygène, d'azote, de silicium, de soufre, par un groupement SO₂, ; un radical onium Z, le radical R₁ ne comportant pas de liaison peroxyde, ni de radicaux diazo, nitro ou nitroso ,
• R₂ représente un radical onium Z choisi parmi les radicaux Z de formule (II), (III) ou (IV) suivante ou un radical -X-C=NR₈-NR₉R₁₀ ou - XP (O) (O-) OCH₂CH₂N⁺(CH₃)₃ dans lequel X représente un atome d'oxygène ou un radical -NR₁₁ et R₈, R₉,R₁₀ et R₁₁ représentent un hydrogène, un radical alkyle en C₁-C₄ ou un radical hydroxyalkyle en C₁-C₄ ;
• Formule (II) 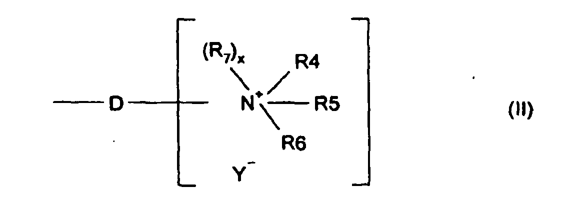
dans laquelle
• D est une liaison covalente ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₈ ou amino, et pouvant porter une ou plusieurs fonctions cétone ;
• liaison covalente R₄ R₅ et R₆, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono-, di- ou tri-substituée par un radical alkyle en C₁-C₄, alkyl(C₁-C₆)carbonyle, amido ou alkyl(C₁-C₆)sulfonyle, à la condition que si D représente une liaison covalente alors R4 représente un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono-, di- ou tri-substituée par un radical alkyle en C₁-C₄, alkyl(C₁-C₆)carbonyle, amido ou alkyl(C₁-C₆)sulfonyle;
• R₄, R₅ et R₆ ensemble, deux à deux, forment, avec l'atome d'azote auxquels ils sont rattachés, un cycle saturé carboné à 4, 5, 6 ou 7 chaînons pouvant contenir un ou plusieurs hétéroatomes, le cycle cationique pouvant être substitué par un atome d'halogène, un radical hydroxyle, un radical alkyle en Ci-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkyl(C₁-C₆)carbonyle, un radical thio, un radical thioalkyle en C₁-C₆, un radical alkyl(C₁-C₆)thio, un radical amino, un radical amino mono- di- ou tri substitué par un radical alkyl(C₁-C₆), alkyl(C₁-C₆)carbonyle, amido ou alkyl(C₁-C₆)sulfonyle;
• R₇ représente un radical alkyle en C₁-C₆ ; un radical monohydroxyalkyle en Ci-Ce ; un radical polyhydroxyalkyle en C₂-C₆; un radical aryle ; un radical benzyle ; un radical aminoalkyle en C₁-C₆ ; un radical aminoalkyle en C₁-C₆ dont l'amine est mono- ou di-subsitué par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₈)sulfonamidoalkyle en C₁-C₆ ;
• x est 0 ou 1,
- lorsque x = 0, alors le bras de liaison est rattaché à l'atome d'azote portant les radicaux R₄ à R₈,
- lorsque x = 1, alors deux des radicaux R₄ à R₆ forment conjointement avec l'atome d'azote auquel ils sont rattachés un cycle saturé à 4, 5, 6 ou 7 chaînons et D est lié à un atome de carbone du cycle saturé,
• Formule (III) 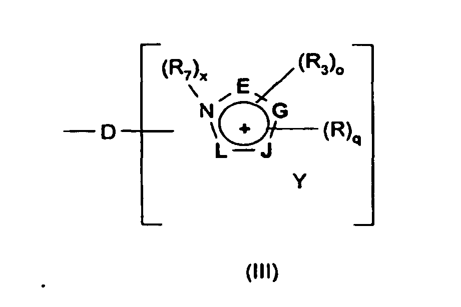
dans laquelle
• D est une liaison covalent ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions cétone;
• les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
• q est un nombre entier compris entre 0 et 4 inclus ;
• o est un nombre entier compris entre 0 et 3 inclus ;
• q+o est un nombre entier compris entre 0 et 4
• R, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆ un radical trialkyl(C₁-C₆)silanealkyle en C₁-C₆ un radical amido, un radical carboxyle, un radical alkylcarbonyle en C₁-C₆, un radical thio, un radical thioalkyle en C₁-C₆, un radical alkyl(C₁-C₆)thio, un radical amino, un radical amino mono ou di substituée par un radical alkyl(C₁-C₆), alkyl(C₁-C₆)carbonyle, amido, alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
• R₃, identique ou différent, représente un radical alkyle en C₁-C₆, un radical monohydroxyalkyle en C₁-C₆ un radical polyhydroxyalkyle en C₂-C₆ un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₃ sont portés par un azote,
• R₇ représente un radical alkyle en C₁-C₆ ; un radical monohydroxyalkyle en C₁-C₆; un radical polyhydroxyalkyle en C₂-C₆ ; un radical aryle ; un radical benzyle ; un radical aminoalkyle en C₁-C₆, un radical aminoalkyle en C₁-C₆ dont l'amine est substitué par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carboxyalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x est 0 ou 1
- lorsque x = 0, le bras de liaison D est rattaché à l'atome d'azote,
- lorsque x = 1, le bras de liaison D est rattaché à l'un des sommets E, G, J ou L,
• Formule (IV) 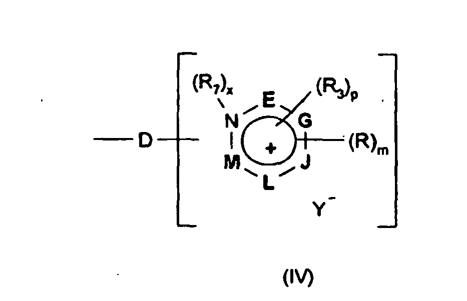
dans laquelle :
• D est une liaison covalent ou une chaîne alkylène en C₁-C₁₄, linéaire ou ramifiée pouvant être interrompue par un ou plusieurs hétéroatomes choisis parmi un atomes d'oxygène, de soufre ou d'azote, et pouvant être substituée par un ou plusieurs radicaux hydroxyle, alcoxy en C₁-C₆ ou amino, et pouvant porter une ou plusieurs fonctions cétone;
• les sommets E, G, J, L et M identiques ou différents, représentent un atome de carbone, d'oxygène, de soufre ou d'azote et forment un cycle choisi parmi les cycles pyridiniques, pyrimidiniques, pyraziniques, triaziniques et pyridaziniques,
• p est un nombre entier compris entre 0 et 3 inclus ;
• m est un nombre entier compris entre 0 et 5 inclus ;
• p+m est un nombre entier compris entre 0 et 5 ;
• R, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thio, un radical thioalkyle en C₁-C₆, un radical alkyl(C₁-C₆)thio, un radical amino, un radical amino substitué par un radical alkyl(C₁-C₆), alkyl(C₁-C₆)carbonyle, amido ou alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
• R₃ , identiques ou différents, représentent un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₃ sont portés par un azote,
• R₇ représente un radical alkyle en C₁-C₆; un radical monohydroxyalkyle en C₁-C₈ ; un radical polyhydroxyalkyle en C₂-C₈; un radical aryle ; un radical benzyle ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C1-C6; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carboxyalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x est 0 ou 1
- lorsque x = 0, le bras de liaison D est rattaché à l'atome d'azote,
- lorsque x = 1, le bras de liaison D est rattaché à l'un des sommets E, G, J, L ou M,
Y⁻ représente un contre ion.

2. Dérivés selon la revendication 1 dans lesquels n est égal à 0.

3. Dérivés selon la revendication 1 dans lesquels n est égal à 1 et R₁ est un atome d'halogène ; une chaîne hydrocarbonée en C₁-C₆, aliphatique ou alicyclique, saturée ou insaturée, un ou plusieurs atomes de carbone pouvant être remplacés par un atome d'oxygène, d'azote, de silicium, de soufre, par un groupement SO₂, le radical R₁ ne comportant pas de liaison peroxyde, ni de radicaux diazo, nitro ou nitroso.

4. Dérivés selon la revendication 1 dans lesquels R₁ est choisi parmi le chlore, le brome, un radical alkyle en C₁-C₄, hydroxyalkyle en C₁-C₄, aminoalkyle en C₁-C₄, alcoxy en C₁-C₄, hydroxyalcoxy en C₁-C₄.

5. Dérivés selon la revendication 4 dans lesquels R₁ est choisi parmi un radical méthyle, hydroxyméthyle, 2-hydroxyéthyle, 1,2-dihydroxyéthyle, méthoxy, isopropyloxy, 2-hydroxyéthoxy.

6. Dérivés selon l'une quelconque des revendications 1 à 5 dans lesquels R₂ de formule (II) est tel que x est égal à 0, et R₅ et R₆ séparément sont choisis parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical amidoalkyle en C₁-C₆, un radical trialkyl(C₁-C₆)silanealkyle en C₁-C₆ , ou R₄ avec R₅ forment ensemble un cycle azétidine, pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle mono- ou di-substitué par un radical alkyl(C₁-C₆), alkyl(C₁-C₆)carbonyle, amido ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamylalkyle en C₁-C₆.

7. Dérivés selon l'une quelconque des revendications 1 à 5 dans lesquels R₂ de formule (II) est tel que x est égal à 1, R₇ est choisi parmi un radical alkyle en Ci-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un alkyl(C₁-C₆)carbonylalkyle en C₁-C₆ ; un radical N-alkyl(C₁-C₆)carbamylalkyle en C₁-C₆ ; R₄ avec R₅ ensemble forment un cycle azétidine pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ : un radical alkyl(C₁-C₆)carbonylalkyle en C₁-C₆ ; un radical N-alkyl(C₁-C₆)carbamylalkyle en C₁-C₆.

8. Dérivés selon l'une quelconque des revendications 1 à 7 dans lesquels D est une liaison covalente ou une chaîne alkylène pouvant être substituée.

9. Dérivés selon la revendication 8 dans lesquels R₂ de formule (III) est tel que les sommets E, G, J et L forment un cycle pyrrolique, imidazolique, pyrazolique, oxazolique, thiazolique et triazolique.

10. Dérivés selon la revendication 9 dans lesquels les sommets E, G, J et L forment un cycle imidazolique.

11. Dérivés selon la revendication 1 dans lesquels R2 de formule (IV) est tel que les sommets E,G,J,L et M avec l'azote du cycle forment un cycle choisi parmi les cycles pyridiniques, pyrimidiniques.

12. Dérivés selon l'une quelconque des revendications 1 à 11 dans lesquels R₂ de formule (III) ou (IV) est tel que R, R₇ et R₃ sont des radicaux alkyles pouvant être substitués.

13. Dérivés selon la revendication 1 dans lesquels R₂ est un radical -X-C=NR₈-NR₉R₁₀, X représente un atome d'oxygène ou un radical -NR₁₁, R₈ R₉ R₁₀ et R₁₁ représentant un hydrogène, un radical alkyle en C₁-C₄ ou un radical hydroxyalkyle.

14. Dérivés selon la revendication 14 dans lesquels X est -NR₁₁, R₈ est un hydrogène, R₉ et R₁₀ sont choisis parmi l'hydrogène ou un radical alkyle.

15. Dérivés selon l'une quelconque des revendications 1 à 14 choisis parmi
- N'-[1-(4-Amino-phényl)-pyrrolidin-3-yl]-N,N-diméthyl-guanidine
- N-[1-(4-Amino-phényl)-pyrrolidin-3-yl]-guanidine
- 3-[1-(4-Amino-phényl)-pyrrolidin-3-yl]-1-méthyl-3H-imidazol-1 -ium; chlorure
- [1-(4-Amino-phényl)-pyrrolidin-3-yl]-diméthyl-(3-triméthylsilanyl-propyl)-ammonium ; chlorure
- N'-[1-(4-Amino-3-méthyl-phényl)-pyrrolidin-3-yl]-N,N-diméthyl-guanidine
- N-[1-(4-Amino-3-méthyl-phényl)-pyrrolidin-3-yl]-guanidine
- 3-[1-(4-Amino-3-méthyl-phényl)-pyrrolidin-3-yl]-1-méthyl-3H-imidazol-1-ium; chlorure
- [1-(4-Amino-3-méthyl-phényl)-pyrrolidin-3-yl]-diméthyl-(3-triméthylsilanyl-propyl ammonium ; chlorure
- 3-[1-(4-Amino-phényl)-pyrrolidin-3-yl]-1-(3-triméthylsilanyl-propyl)-3H-imidazol-1-ium ; chlorure
- 3-[1-(4-Amino-3-méthyl-phényl)-pyrrolidin-3-yl]-1-(3-triméthylsilanyl-propyl)-3H-imidazol-1-ium ; chlorure
- 1'-(4-Amino-phényl)-1-méthyl-[1,3']bipyrrolidinyl-1-ium; chlorure
- 1'-(4-Amino-3-méthyl-phényl)-1 -méthyl-[1,3']bipyrrolidinyl-1 -ium; chlorure
- -3-{[1-(4-Amino-phényl)-pyrrolidin-3-ylcarbamoyl]-méthyl}-1-méthyl-3H-imidazol-1-ium ; chlorure
- -3-{[1-(4-Amino-3-méthyl-phényl)-pyrrolidin-3-ylcarbamoyl]-méthyl}-1-méthyl-3H-imidazol-1-ium ; chlorure

16. Dérivés selon la revendication 15 choisis parmi le 1-méthyl-3-[1-(4-amino-phényl)-pyrrolidin-3-yl]-3H-imidazol-1-ium , chlorure ; chlorhydrate, le N-[1-(4-Amino-phényl)-pyrrolidin-3-yl]-guaninidine ; trichlorhydrate et le l'-(4-Amino-phényl)-1-méthyl-[1,3']bipyrrolidinyl-1-ium; chlorure

17. Dérivés selon la revendication 1 dans lesquels la chaîne D comprend un radical phosphoryle.

18. Dérivés selon la revendication 17 choisis parmi le [1-(4-Amino-phényl)-pyrrolidin-3-yl]-oxophosphorylcholine, le [1-(4-Amino-3-méthyl-phényl)-pyrrolidin-3-yl]-oxophosphorylcholine.

19. Composition tinctoriale comprenant à titre de base d'oxydation au moins un dérivé paraphénylènediamine de formule (I), tel que défini selon l'une quelconque des revendications 1 à 18.

20. Composition selon la revendication 19 comprenant de plus un coupleur choisi parmi les méta-phénylènediamines, les méta-aminophénols, les méta-diphénols, les coupleurs naphtaléniques, les coupleurs hétérocycliques et leurs sels d'addition

21. Composition selon l'une quelconque des revendications 19 ou 20 comprenant une base d'oxydation additionnelle autre que les bases d'oxydation de formule (I) choisie parmi les para-phénylènediamines, les bis-phénylalkylènediamines, les para-aminophénols, les ortho-aminophénols, les bases hétérocycliques et leurs sels d'addition.

22. Composition selon l'une quelconque des revendications précédentes dans laquelle la quantité de chacune des bases d'oxydation est comprise entre 0,001 à 10 % en poids environ du poids total de la composition tinctoriale.

23. Composition selon la revendication 22 dans laquelle la quantité de chacun des coupleurs est comprise entre 0,001 et 10 % en poids environ du poids total de la composition tinctoriale.

24. Procédé de teinture d'oxydation des fibres kératiniques, **caractérisé en ce qu'**on applique sur les fibres une composition tinctoriale telle que définie dans l'une quelconque des revendications 19 à 23 en présence d'un agent oxydant pendant un temps suffisant pour développer la coloration désirée.

25. Procédé selon la revendication 24 dans lequel l'agent oxydant est choisi parmi le peroxyde d'hydrogène, le peroxyde d'urée, les bromates de métaux alcalins, les persels, les peracides et les enzymes oxydases.

26. Dispositif à plusieurs compartiments dans lequel un premier compartiment contient une composition tinctoriale telle que définie à l'une quelconque des revendications 20 à 24 et un deuxième compartiment contient un agent oxydant.

27. Utilisation de la composition définie aux revendications 19 à 23 pour la teinture de fibres kératiniques.

28. Composés nitro de formule (I') suivante 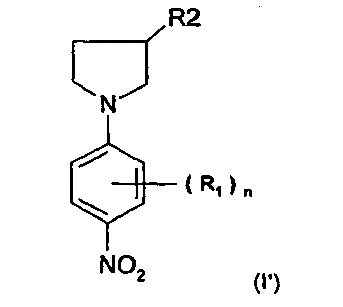 dans laquelle R₁, n et R₂ sont tels que définis selon l'une quelconque des revendications 1 à 21.

## Claims

1. Para-phenylenediamine derivatives substituted with a pyrrolidinyl group of formula (I), and the addition salts thereof 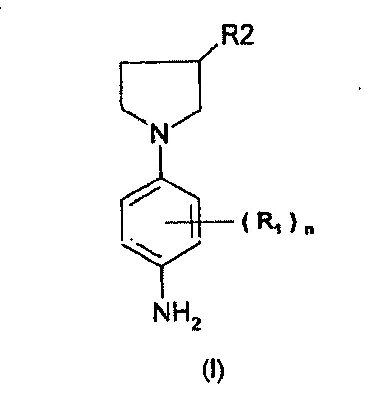 in which
• n is between 0 and 4, it being understood that when n is greater than or equal to 2, then the radicals R₁ may be identical or different,
• R₁ represents a halogen atom; a C₁-C₆ aliphatic or alicyclic, saturated or unsaturated hydrocarbon-based chain, one or more carbon atoms possibly being replaced with an oxygen, nitrogen, silicon or sulfur atom or an SO₂ group; an onium radical Z; the radical R₁ not comprising a peroxide bond or diazo, nitro or nitroso radicals,
• R₂ represents an onium radical Z chosen from the radicals Z of formula (II), (III) or (IV) below or a radical -X-C=NR₈-NR₉R₁₀ or -XP (O) (O-)OCH₂CH₂N⁺(CH₃)₃ in which X represents an oxygen atom or a radical -NR₁₁ and R₈, R₉, R₁₀ and R₁₁ represent a hydrogen atom or a C₁-C₄ alkyl radical or a C₁-C₄ hydroxyalkyl radical;
• Formula (II) 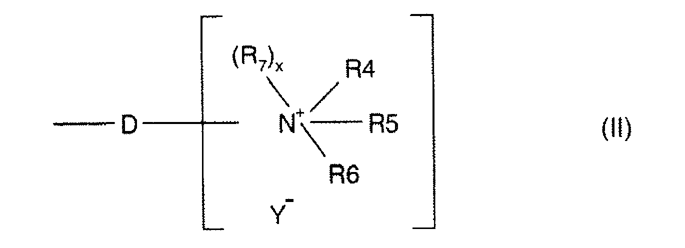
in which
• D is a covalent bond or a linear or branched C₁-C₁₄ alkylene chain which may be interrupted with one or more hetero atoms chosen from oxygen, sulfur and nitrogen, and which may be substituted with one or more hydroxyl, C₁-C₆ alkoxy or amino radicals, and which may bear one or more ketone functions;
• R₄, R₅ and R₆, taken separately, represent a C₁-C₁₅ alkyl radical; a C₁-C₆ monohydroxyalkyl radical; a C₂-C₆ polyhydroxyalkyl radical; a (C₁-C₆) alkoxy-(C₁-C₆)alkyl radical; an aryl radical; a benzyl radical; a C₁-C₆ amidolalkyl radical; a tri (C₁-C₆) - alkylsilane (C₁-C₆) alkyl radical; a C₁-C₆ aminoalkyl radical; a C₁-C₆ aminoalkyl radical in which the amine is mono-, di- or trisubstituted with a C₁-C₄ alkyl, (C₁-C₆)alkylcarbonyl, amido or (C₁-C₆)alkylsulfonyl radical; with the proviso that when D represents a covalent bond, then R4 represents an aryl radical;
• R₄, R₅ and R₆ together, in pairs, form, with the nitrogen atom to which they are attached, a saturated 4-, 5-, 6- or 7-membered carbon-based ring optionally containing one or more hetero atoms, the cationic ring possibly being substituted with a halogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri (C₁-C₆) alkylsilane(C₁-C₆)alkyl radical, an amido radical, a carboxyl radical, a (C₁-C₆)alkylcarbonyl radical, a thio radical, a C₁-C₆ thioalkyl radical, a (C₁-C₆)alkylthio radical, an amino radical, an amino radical mono-, di- or trisubstituted with a (C₁-C₆) alkyl, (C₁-C₆) alkylcarbonyl, amido or (C₁-C₆)alkylsulfonyl radical;
• R₇ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical; a C₁-C₆ aminoalkyl radical in which the amine is mono- or disubstituted with a (C₁-C₆) alkyl, (C₁-C₆) alkylcarbonyl, amido or (C₁-C₆) alkylsulfonyl radical; a C₁-C₆ carboxyalkyl radical; a C₁-C₆ carbamylalkyl radical; a C₁-C₆ trifluoroalkyl radical; a tri (C₁-C₆) alkylsilane (C₁-C₆) - alkyl radical; a C₁-C₆ sulfonamidoalkyl radical; a (C₁-C₆) alkylcarboxy(C₁-C₆) alkyl radical; a (C₁-C₆) - alkylsulfinyl (C₁-C₆) alkyl radical; a (C₁-C₆) - alkylsulfonyl(C₁-C₆)alkyl radical; a (C₁-C₆) alkylcarbonyl (C₁-C₆) alkyl radical; an N-(C₁-C₆) alkylcarbamyl (C₁-C₆) alkyl radical; an N-(C₁-C₆) alkylsulfonamido (C₁-C₆) alkyl radical;
• x is 0 or 1,
- when x = 0, then the linker arm is attached to the nitrogen atom bearing the radicals R₄ to R₆,
- when x = 1, then two of the radicals R₄ to R₆ form, together with the nitrogen atom to which they are attached, a 4-, 5-, 6- or 7-membered saturated ring and D is linked to a carbon atom of the saturated ring;
• Formula (III) 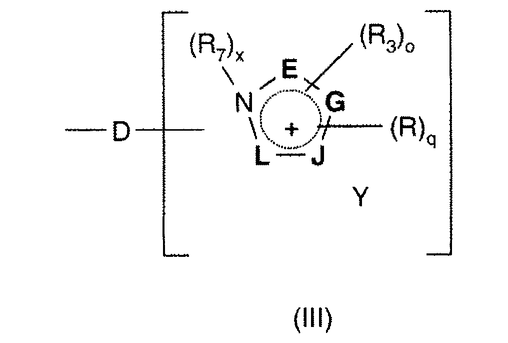
in which
• D is a covalent bond or a linear or branched C₁-C₁₄ alkylene chain that may be interrupted with one or more hetero atoms chosen from oxygen, sulfur and nitrogen, and that may be substituted with one or more hydroxyl, C₁-C₆ alkoxy or amino radicals, and that may bear one or more ketone functions;
• the ring members E, G, J and L, which may be identical or different, represent a carbon, oxygen, sulfur or nitrogen atom to form a pyrrole, pyrazole, imidazole, triazole, oxazole, isoxazole, thiazole or isothiazole ring,
• q is an integer between 0 and 4 inclusive;
• o is an integer between 0 and 3 inclusive;
• q+o is an integer between 0 and 4;
• R, which may be identical or different, represent a halogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri(C₁-C₆)alkylsilane(C₁-C₆) alkyl radical, an amido radical, a carboxyl radical, a C₁-C₆ alkylcarbonyl radical, a thio radical, a C₁-C₆ thioalkyl radical, a (C₁-C₆) alkylthio radical, an amino radical, an amino radical mono- or disubstituted with a (C₁-C₆) alkyl, (C₁-C₆) alkylcarbonyl, amido or (C₁-C₆) alkylsulfonyl radical; a C₁-C₆ monohydroxyalkyl radical or a C₂-C₆ polyhydroxyalkyl radical; it being understood that the radicals R are borne by a carbon atom,
• R₃, which may be identical or different, represent a C₁-C₆ alkyl radical, a C₁-C₆ monohydroxyalkyl radical, a C₂-C₆ polyhydroxyalkyl radical, a tri (C₁-C₆) alkylsilane (C₁-C₆) alkyl radical, a (C₁-C₆) alkoxy(C₁-C₆) alkyl radical, a C₁-C₆ carbamylalkyl radical, a (C₁-C₆) alkylcarboxy (C₁-C₆) alkyl radical or a benzyl radical; it being understood that the radicals R₃ are borne by a nitrogen atom,
• R₇ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is substituted with a (C₁-C₆)alkyl, (C₁-C₆)alkylcarbonyl, amido or (C₁-C₆) alkylsulfonyl radical; a C₁-C₆ carboxyalkyl radical; a C₁-C₆ carbamylalkyl radical; a C₁-C₆ trifluoroalkyl radical; a tri (C₁-C₆) alkylsilane (C₁-C₆) alkyl radical; a C₁-C₆ sulfonamidoalkyl radical; a (C₁-C₆) alkylcarboxy(C₁-C₆) alkyl radical; a (C₁-C₆) - alkylsulfinyl (C₁-C₆) alkyl radical; a (C₁-C₆) alkylsulfonyl (C₁-C₆) alkyl radical; a (C₁-C₆) alkylcarboxy (C₁-C₆) alkyl radical; an N- (C₁-C₆) alkylcarbamyl (C₁-C₆) alkyl radical; an N- (C₁-C₆) alkylsulfonamido (C₁-C₆) alkyl radical;
• x is 0 or 1
- when x = 0, the linker arm D is attached to the nitrogen atom,
- when x = 1, the linker arm D is attached to one of the ring members E, G, J or L,
• Formula (IV) 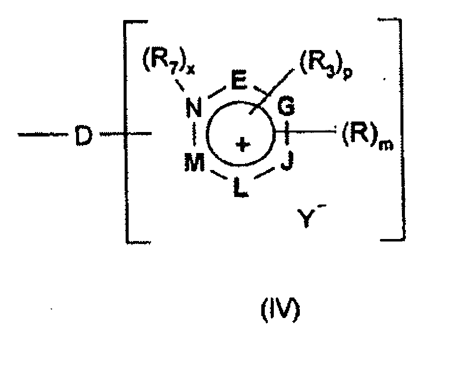
in which:
• D is a covalent bond or a linear or branched C₁-C₁₄ alkylene chain which may be interrupted with one or more hetero atoms chosen from oxygen, sulfur and nitrogen atoms, and which may be substituted with one or more hydroxyl, C₁-C₆ alkoxy or amino radicals, and which may bear one or more ketone functions;
• the ring members E, G, J, L and M, which may be identical or different, represent a carbon, oxygen, sulfur or nitrogen atom and form a ring chosen from pyridine, pyrimidine, pyrazine, triazine and pyridazine rings;
• p is an integer between 0 and 3 inclusive;
• m is an integer between 0 and 5 inclusive;
• p+m is an integer between 0 and 5;
• R, which may be identical or different, represent a halogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri (C₁-C₆) alkylsilane (C₁-C₆) alkyl radical, an amido radical, a carboxyl radical, a C₁-C₆ alkylcarbonyl radical, a thio radical, a C₁-C₆ thioalkyl radical, a (C₁-C₆) alkylthio radical, an amino radical, an amino radical substituted with a (C₁-C₆) alkyl, (C₁-C₆) alkylcarbonyl, amido or (C₁-C₆) alkylsulfonyl radical; a C₁-C₆ monohydroxyalkyl radical or a C₂-C₆ polyhydroxyalkyl radical; it being understood that the radicals R are borne by a carbon atom,
• R₃, which may be identical or different, represent a C₁-C₆ alkyl radical, a C₁-C₆ monohydroxyalkyl radical, a C₂-C₆ polyhydroxyalkyl radical, a tri (C₁-C₆) alkylsilane (C₁-C₆) alkyl radical, a (C₁-C₆) alkoxy(C₁-C₆) alkyl radical, a C₁-C₆ carbamylalkyl radical, a (C₁-C₆) alkylcarboxy (C₁-C₆) alkyl radical or a benzyl radical; it being understood that the radicals R₃ are borne by a nitrogen atom,
• R₇ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is mono- or disubstituted with a (C₁-C₆) alkyl, (C₁-C₆) alkylcarbonyl, amido or (C₁-C₆) alkylsulfonyl radical; a C₁-C₆ carboxyalkyl radical; a C₁-C₆ carbamylalkyl radical; a C₁-C₆ trifluoroalkyl radical; a tri (C₁-C₆) alkylsilane (C₁-C₆) alkyl radical; a C₁-C₆ sulfonamidoalkyl radical; a (C₁-C₆)alkylcarboxy(C₁-C₆) alkyl radical; a (C₁-C₆) alkylsulfinyl (C₁-C₆) alkyl radical; a (C₁-C₆) alkylsulfonyl (C₁-C₆) alkyl radical; a (C₁-C₆) alkylcarboxy (C₁-C₆) alkyl radical; an N- (C₁-C₆) alkylcarbamyl (C₁-C₆) alkyl radical; an N- (C₁-C₆) alkylsulfonamido (C₁-C₆) alkyl radical;
• x is 0 or 1
- when x = 0, the linker arm D is attached to the nitrogen atom,
- when x = 1, the linker arm D is attached to one of the ring members E, G, J, L or M,
• Y⁻ is a counterion.

2. Derivatives according to Claim 1, in which n is equal to 0.

3. Derivatives according to Claim 1, in which n is equal to 1 and R₁ is a halogen atom; a saturated or unsaturated, aliphatic or alicyclic C₁-C₆ hydrocarbon-based chain, one or more carbon atoms possibly being replaced with an oxygen, nitrogen, silicon or sulfur atom or with a group SO₂, the radical R₁ not comprising a peroxide bond or any diazo, nitro or nitroso radicals.

4. Derivatives according to Claim 1, in which R₁ is chosen from chlorine or bromine, a C₁-C₄ alkyl, C₁-C₄ hydroxyalkyl, C₁-C₄ aminoalkyl, C₁-C₄ alkoxy or C₁-C₄ hydroxyalkoxy radical.

5. Derivatives according to Claim 4, in which R₁ is chosen from a methyl, hydroxymethyl, 2-hydroxyethyl, 1,2-dihydroxyethyl, methoxy, isopropyloxy or 2-hydroxyethoxy radical.

6. Derivatives according to any one of Claims 1 to 5, in which R₂ of formula (II) is such that x is equal to 0, and R₅ and R₆, separately, are chosen from a C₁-C₆ alkyl radical, a C₁-C₄ monohydroxyalkyl radical, a C₂-C₄ polyhydroxyalkyl radical, a (C₁-C₆) alkoxy (C₁-C₄) alkyl radical, a C₁-C₆ amidoalkyl radical, a tri (C₁-C₆) alkylsilane (C₁-C₆) alkyl radical, or R₄ and R₅ together form an azetidine, pyrrolidine, piperidine, piperazine or morpholine ring, R₆ being chosen in this case from a C₁-C₆ alkyl radical; a C₁-C₆ monohydroxyalkyl radical; a C₂-C₆ polyhydroxyalkyl radical; a C₁-C₆ aminoalkyl radical, a C₁-C₆ aminoalkyl radical mono- or disubstituted with a (C₁-C₆)alkyl, (C₁-C₆) alkylcarbonyl, amido or (C₁-C₆) alkylsulfonyl radical; a C₁-C₆ carbamoylalkyl radical; a tri (C₁-C₆) alkylsilane (C₁-C₆) alkyl radical; a (C₁-C₆) alkylcarboxy (C₁-C₆) alkyl radical; a (C₁-C₆) alkylcarbonyl (C₁-C₆) alkyl radical; an N- (C₁-C₆) alkylcarbamyl (C₁-C₆) alkyl radical.

7. Derivatives according to any one of Claims 1 to 5, in which R₂ of formula (II) is such that x is equal to 1 and R₇ is chosen from a C₁-C₆ alkyl radical; a C₁-C₆ monohydroxyalkyl radical; a C₂-C₆ polyhydroxyalkyl radical; a C₁-C₆ aminoalkyl radical; a C₁-C₆ aminoalkyl radical in which the amine is mono- or disubstituted with a (C₁-C₆) alkyl, (C₁-C₆) alkylcarbonyl, amido or (C₁-C₆) alkylsulfonyl radical; a C₁-C₆ carbamylalkyl radical; a tri (C₁-C₆) alkylsilane (C₁-C₆) alkyl radical; a (C₁-C₆) alkylcarboxy (C₁-C₆) alkyl radical; a (C₁-C₆) alkylcarbonyl (C₁-C₆) alkyl radical; an N- (C₁-C₆) alkylcarbamoyl (C₁-C₆) alkyl radical; R₄ and R₅ together form an azetidine, pyrrolidine, piperidine, piperazine or morpholine ring, R₆ being chosen in this case from a C₁-C₆ alkyl radical; a C₁-C₆ monohydroxyalkyl radical; a C₂-C₆ polyhydroxyalkyl radical; a C₁-C₆ aminoalkyl radical, a C₁-C₆ aminoalkyl radical in which the amine is mono- or disubstituted with a (C₁-C₆)alkyl, (C₁-C₆) alkylcarbonyl, amido or (C₁-C₆) alkylsulfonyl radical; a C₁-C₆ carbamylalkyl radical; a tri-(C₁-C₆)alkylsilane(C₁-C₆)alkyl radical; a (C₁-C₆) alkylcarboxy (C₁-C₆) alkyl radical; a (C₁-C₆) alkylcarbonyl-(C₁-C₆) alkyl radical; an N- (C₁-C₆) alkylcarbamyl-(C₁-C₆) alkyl radical.

8. Derivatives according to any one of Claims 1 to 7, in which D is a covalent bond or an alkylene chain that may be substituted.

9. Derivatives according to Claim 8, in which R₂ of formula (III) is such that the ring members E, G, J and L form a pyrrole, imidazole, pyrazole, oxazole, thiazole or triazole ring.

10. Derivatives according to Claim 9, in which the ring members E, G, J and L form an imidazole ring.

11. Derivatives according to Claim 1, in which R₂ of formula (IV) is such that the ring members E, G, J, L and M form with the nitrogen of the ring a ring chosen from pyridine and pyrimidine rings.

12. Derivatives according to any one of Claims 1 to 11, in which R₂ of formula (III) or (IV) is such that R, R₇ and R₃ are alkyl radicals that may be substituted.

13. Derivatives according to Claim 1, in which R₂ is a radical -X-C=NR₈-NR₉R₁₀ and X represents an oxygen atom or a radical -NR₁₁, R₈, R₉, R₁₀ and R₁₁ representing a hydrogen atom or a C₁-C₄ alkyl or hydroxyalkyl radical.

14. Derivatives according to Claim 13, in which X is -NR₁₁, R₈ is a hydrogen, and R₉ and R₁₀ are chosen from hydrogen or an alkyl radical.

15. Derivatives according to any one of Claims 1 to 14, chosen from
- N'-[1-(4-aminophenyl)pyrrolidin-3-yl]-N,N-dimethylguanidine
- N-[1-(4-aminophenyl)pyrrolidin-3-yl]guanidine
- 3-[1-(4-aminophenyl)pyrrolidin-3-yl]-1-methyl-3H-imidazol-1-ium chloride
- [1-(4-aminophenyl)pyrrolidin-3-yl]dimethyl(3-trimethylsilanylpropyl)ammonium chloride
- N'-[1-(4-amino-3-methylphenyl)pyrrolidin-3-yl]-N,N-dimethylguanidine
- N-[1-(4-amino-3-methylphenyl)pyrrolidin-3-yl]-guanidine
- 3-[1-(4-amino-3-methylphenyl)pyrrolidin-3-yl]-1-methyl-3H-imidazol-1-ium chloride
- [1-(4-amino-3-methylphenyl)pyrrolidin-3-yl]-dimethyl(3-trimethylsilanylpropyl)ammonium chloride
- 3-[1-(4-aminophenyl)pyrrolidin-3-yl]-1-(3-trimethylsilanylpropyl)-3H-imidazol-1-ium chloride
- 3-[1-(4-amino-3-methylphenyl)pyrrolidin-3-yl]-1-(3-trimethylsilanylpropyl)-3H-imidazol-1-ium chloride
- 1'-(4-aminophenyl)-1-methyl[1,3']bipyrrolidinyl-1-ium chloride
- 1'-(4-amino-3-methylphenyl)-1-methyl[1,3']/bipyrrolidinyl-1-ium chloride
- -3-{[1-(4-aminophenyl)pyrrolidin-3-ylcarbamoyl]-methyl}-1-methyl-3H-imidazol-1-ium chloride
- -3-{[1-(4-amino-3-methylphenyl)pyrrolidin-3-yl-carbamoyl]methyl}-1-methyl-3H-imidazol-1-ium chloride.

16. Derivatives according to Claim 15, chosen from 1-methyl-3-[1-(4-aminophenyl)pyrrolidin-3-yl]-3H-imidazol-1-ium chloride hydrochloride; N-[1-(4-aminophenyl)pyrrolidin-3-yl]guanidine trihydrochloride and 1'-(4-aminophenyl)-1-methyl[1,3']bipyrrolidinyl-1-ium chloride.

17. Derivatives according to Claim 1, in which the chain D comprises a phosphoryl radical.

18. Derivatives according to Claim 17, chosen from [1-(4-aminophenyl)pyrrolidin-3-yl]oxophosphorylcholine and [1-(4-amino-3-methylphenyl)-pyrrolidin-3-yl]oxophosphorylcholine.

19. Dye composition comprising, as oxidation base, at least one para-phenylenediamine derivative of formula (I), as defined in any one of Claims 1 to 18.

20. Composition according to Claim 19, further comprising a coupler chosen from meta-phenylenediamines, meta-aminophenols, meta-diphenols, naphthalenic couplers and heterocyclic couplers, and the addition salts thereof.

21. Composition according to either of Claims 19 and 20, comprising an additional oxidation base other than the oxidation bases of formula (I), chosen from para-phenylenediamines, bis(phenyl)-alkylenediamines, para-aminophenols, ortho-aminophenols and heterocyclic bases, and the addition salts thereof.

22. Composition according to any one of the preceding claims, in which the amount of each of the oxidation bases is between 0.001% and 10% by weight approximately relative to the total weight of the dye composition.

23. Composition according to Claim 22, in which the amount of each of the couplers is between 0.001% and 10% by weight approximately relative to the total weight of the dye composition.

24. Process for the oxidation dyeing of keratin fibres, **characterized in that** a dye composition as defined in any one of Claims 19 to 23 is applied to the fibres in the presence of an oxidizing agent, for a time that is sufficient to develop the desired coloration.

25. Process according to Claim 24, in which the oxidizing agent is chosen from hydrogen peroxide, urea peroxide, alkali metal bromates, persalts, peracids and oxidase enzymes.

26. Multi-compartment device in which a first compartment contains a dye composition as defined in any one of Claims 19 to 23 and a second compartment contains an oxidizing agent.

27. Use of the composition defined in Claims 19 to 23 for dyeing keratin fibres.

28. Nitro compounds of formula (I') below 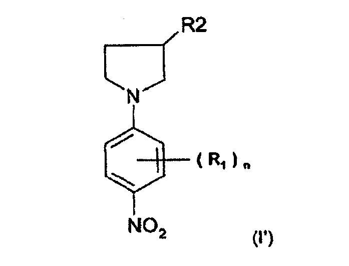 in which R₁, n and R₂ are as defined according to any one of Claims 1 to 21.

## Patentansprüche

1. Durch eine Pyrrolidinylgruppe substituierte para-Phenylendiaminderivate der Formel (I) und ihre Säureadditionssalze 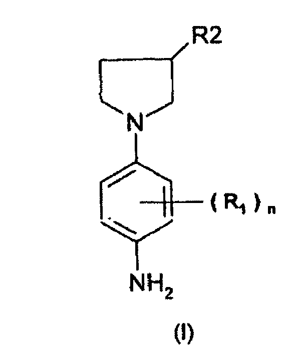 worin
• n zwischen 0 und 4 liegt, mit der Maßgabe, daß dann, wenn n größer gleich 2 ist, die Reste R₁ gleich oder verschieden sein können,
• R₁ für ein Halogenatom; eine gesättigte oder ungesättigte, aliphatische oder alicyclische C₁-C₆-Kohlenwasserstoffkette, wobei ein oder mehrere Kohlenstoffatome durch ein Sauerstoff-, Stickstoff-, Silicium- oder Schwefelatom oder eine SO₂-Gruppe ersetzt sein können; oder einen Oniumrest Z steht, wobei der Rest R₁ weder eine Peroxidbindung noch Diazo-, Nitro- oder Nitrosoreste enthält;
• R₂ für einen Oniumrest Z, der unter den Resten Z der folgenden Formel (II), (III) oder (IV) ausgewählt ist, oder einen -X-C=NR₈-NR₉R₁₀- oder -XP (O) (O-)OCH₂CH₂N⁺(CH₃)₃-Rest, worin X für ein Sauerstoffatom oder einen -NR₁₁-Rest steht und R₈, R₉, R₁₀ und R₁₁ für Wasserstoff, einen C₁-C₄-Alkylrest oder einen C₁-C₄-Hydroxyalkylrest stehen, steht;
• Formel (II) 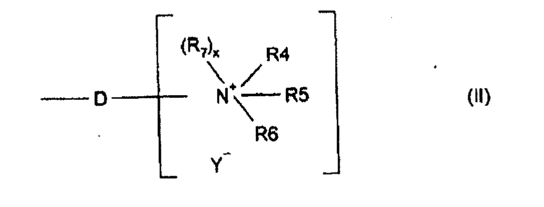
worin
• D für eine kovalente Bindung oder eine lineare oder verzweigte C₁- C₁₄-Alkylenkette, die durch ein oder mehrere unter Sauerstoff, Schwefel oder Stickstoff ausgewählte Heteroatome unterbrochen sein kann und durch einen oder mehrere Hydroxyl-, C₁-C₆-Alkoxy- oder Aminoreste substituiert sein kann und eine oder mehrere Ketonfunktionen tragen kann, steht;
• R₄, R₅ und R₆ separat genommen für einen C₁-C₁₅-Alkylrest; einen C₁-C₆-Monohydroxyalkylrest; einen C₂-C₆-Polyhydroxyalkylrest; einen C₁-C₆-Alkoxy-C₁-C₆-alkylrest; einen Arylrest; einen Benzylrest; einen C₁-C₆-Amidoalkylrest; einen Tri-C₁-C₆-alkylsilan-C₁-C₆-alkylrest; einen C₁-C₆-Aminoalkylrest oder einen C₁-C₆-Aminoalkylrest, dessen Amin ein-, zwei- oder dreifach durch einen C₁-C₄-Alkyl-, C₁-C₆-Alkylcarbonyl-, Amido- oder C₁-C₆-Alkylsulfonylrest substituiert ist; stehen; mit der Maßgabe, daß dann, wenn D für eine kovalente Bindung steht, R4 für einen Arylrest; einen Benzylrest; einen C₁-C₆-Amidoalkylrest; einen Tri-C₁-C₆-alkylsilan-C₁-C₆-alkylrest; einen C₁-C₆-Aminoalkylrest oder einen C₁-C₆-Aminoalkylrest, dessen Amin ein-, zwei-oder dreifach durch einen C₁-C₄-Alkyl-, C₁-C₆-Alkylcarbonyl-, Amido- oder C₁-C₆-Alkylsulfonylrest substituiert ist; steht;
• R₄, R₅ und R₆ zusammen paarweise mit dem Stickstoffatom, an das sie gebunden sind, einen gesättigten 4-, 5-, 6- oder 7-gliedrigen Kohlenstoffring, der ein oder mehrere Heteroatome enthalten kann, bilden, wobei der kationische Ring durch ein Halogenatom, einen Hydroxylrest, einen C₁-C₆-Alkylrest, einen C₁-C₆-Monohydroxyalkylrest, einen C₂-C₆-Polyhydroxyalkylrest, einen C₁-C₆-Alkoxyrest, einen Tri-C₁-C₆-alkylsilan-C₁-C₆-alkylrest, einen Amidorest, einen Carboxylrest, einen C₁-C₆-Alkylcarbonylrest, einen Thiorest, einen C₁-C₆-Thioalkylrest, einen C₁-C₆-Alkylthiorest, einen Aminorest oder einen Aminorest, der ein-, zwei-oder dreifach durch einen C₁-C₆-Alkyl-, C₁-C₆-Alkylcarbonyl-, Amido- oder C₁-C₆-Alkylsulfonylrest substituiert ist, substituiert sein kann;
• R₇ für einen C₁-C₆-Alkylrest; einen C₁-C₆-Mono-hydroxyalkylrest; einen C₂-C₆-Polyhydroxyalkyl-rest; einen Arylrest; einen Benzylrest; einen C₁-C₆-Aminoalkylrest; einen C₁-C₆-Aminoalkylrest, dessen Amin ein- oder zweifach durch einen C₁-C₆-Alkyl-, C₁-C₆-Alkylcarbonyl-, Amido- oder C₁-C₆-Alkylsulfonylrest substituiert ist; einen C₁-C₆-Carboxyalkylrest; einen C₁-C₆-Carbamylalkyl-rest; einen C₁-C₆-Trifluoralkylrest; einen Tri-C₁-C₆-alkylsilan-C₁-C₆-alkylrest; einen C₁-C₆-Sulfonamidoalkylrest; einen C₁-C₆-Alkylcarboxy-C₁-C₆-alkylrest; einen C₁-C₆-Alkylsulfinyl-C₁-C₆-alkylrest; einen C₁-C₆-Alkylsulfonyl-C₁-C₆-alkylrest; einen C₁-C₆-Alkylcarbonyl-C₁-C₆-alkylrest, einen N-C₁-C₆-Alkylcarbamyl-C₁-C₆-alkylrest oder einen N-C₁-C₆-Alkylsulfonamido-C₁-C₆-alkylrest steht;
• x für 0 oder 1 steht,
- im Fall von x = 0 der Linkerarm an das die Reste R₄ bis R₆ tragende Stickstoffatom gebunden ist,
- im Fall von x = 1 zwei der Reste R₄ bis R₆ zusammen mit dem Stickstoffatom, an das sie gebunden sind, einen 4-, 5-, 6- oder 7-gliedrigen gesättigten Ring bilden und D an ein Kohlenstoffatom des gesättigten Rings gebunden ist,
• Formel (III) 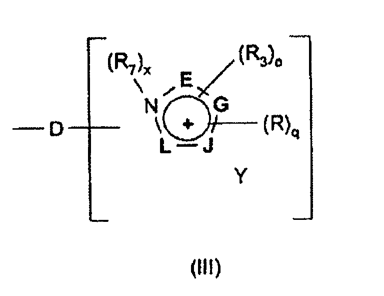
worin
• D für eine kovalente Bindung oder eine lineare oder verzweigte C₁-C₁₄-Alkylenkette, die durch ein oder mehrere unter Sauerstoff, Schwefel oder Stickstoff ausgewählte Heteroatome unterbrochen sein kann und durch einen oder mehrere Hydroxyl-, C₁-C₆-Alkoxy- oder Aminoreste substituiert sein kann und eine oder mehrere Ketonfunktionen tragen kann, steht;
• die Ringglieder E, G, J und L gleich oder verschieden sind und für ein Kohlenstoff-, Sauerstoff-, Schwefel- oder Stickstoffatom zur Bildung eines Pyrrol-, Pyrazol-, Imidazol-, Triazol-, Oxazol-, Isoxazol-, Thiazol- oder Isothiazolrings stehen;
• q für eine ganze Zahl zwischen 0 und 4 einschließlich steht;
• o für eine ganze Zahl zwischen 0 und 3 einschließlich steht;
• q+o für eine ganze Zahl zwischen 0 und 4 steht;
• R gleich oder verschieden ist und für ein Halogenatom, einen Hydroxylrest, einen C₁-C₆-Alkylrest, einen C₁-C₆-Monohydroxyalkylrest, einen C₂-C₆-Polyhydroxyalkylrest, einen C₁-C₆-Alkoxyrest, einen Tri-C₁-C₆-alkylsilan-C₁-C₆-alkylrest, einen Amidorest, einen Carboxylrest, einen C₁-C₆-Alkylcarbonylrest, einen Thiorest, einen C₁-C₆-Thioalkylrest, einen C₁-C₆-Alkyl-thiorest, einen Aminorest, einen Aminorest, der ein- oder zweifach durch einen C₁-C₆-Alkyl-, C₁-C₆-Alkylcarbonyl-, Amido- oder C₁-C₆-Alkylsulfonylrest substituiert ist, steht; mit der Maßgabe, daß die Reste R an einem Kohlenstoffatom stehen;
• R₃ gleich oder verschieden ist und für einen C₁-C₆-Alkylrest, einen C₁-C₆-Monohydroxyalkylrest, einen C₂-C₆-Polyhydroxyalkylrest, einen Tri-C₁-C₆-alkylsilan-C₁-C₆-alkylrest, einen C₁-C₆-Alkoxy-C₁-C₆-alkylrest, einen C₁-C₆-Carbamyl-alkylrest, einen C₁-C₆-Alkylcarboxy-C₁₋₆-alkylrest oder einen Benzylrest steht; mit der Maßgabe, daß die Reste R₃ an einem Stickstoffatom stehen;
• R₇ für einen C₁-C₆-Alkylrest; einen C₁-C₆-Mono-hydroxyalkylrest; einen C₂-C₆-Polyhydroxyalkyl-rest; einen Arylrest; einen Benzylrest; einen C₁-C₆-Aminoalkylrest; einen C₁-C₆-Aminoalkylrest, dessen Amin durch einen C₁-C₆-Alkyl-, C₁-C₆-Alkylcarbonyl-, Amido- oder C₁-C₆-Alkylsulfonylrest substituiert ist; einen C₁-C₆-Carboxyalkylrest; einen C₁-C₆-Carbamylalkylrest; einen C₁-C₆-Trifluoralkylrest; einen Tri-C₁-C₆-alkylsilan-C₁-C₆-alkylrest; einen C₁-C₆-Sulfonamidoalkylrest; einen C₁-C₆-Alkylcarboxy-C₁-C₆-alkylrest; einen C₁-C₆-Alkylsulfinyl-C₁-C₆-alkylrest; einen C₁-C₆-Alkylsulfonyl-C₁-C₆-alkylrest; einen C₁-C₆-Alkylcarboxy-C₁-C₆-alkylrest, einen N-C₁-C₆-Alkylcarbamyl-C₁-C₆-alkylrest oder einen N-C₁-C₆-Alkylsulfonamido-C₁-C₆-alkylrest steht;
• x für 0 oder 1 steht,
- im Fall von x = 0 der Linkerarm D an das Stickstoffatom gebunden ist,
- im Fall von x = 1 der Linkerarm D an eines der Ringglieder E, G, J oder L gebunden ist,
• Formel (IV) 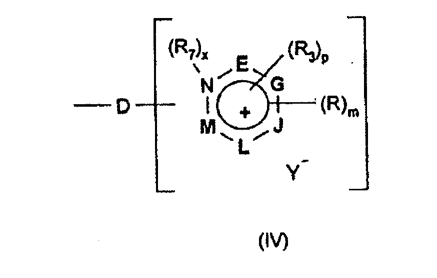
worin
• D für eine kovalente Bindung oder eine lineare oder verzweigte C₁-C₁₄-Alkylenkette, die durch ein oder mehrere unter einem Sauerstoff-, Schwefel- oder Stickstoffatom ausgewählte Heteroatome unterbrochen sein kann und durch einen oder mehrere Hydroxyl-, C₁-C₆-Alkoxy- oder Aminoreste substituiert sein kann und eine oder mehrere Ketonfunktionen tragen kann, steht;
• die Ringglieder E, G, J, L und M gleich oder verschieden sind, für ein Kohlenstoff-, Sauerstoff-, Schwefel- oder Stickstoffatom stehen und einen unter Pyridin-, Pyrimidin-, Pyrazin-, Triazin- und Pyridazinringen ausgewählten Ring bilden;
• p für eine ganze Zahl zwischen 0 und 3 einschließlich steht;
• m für eine ganze Zahl zwischen 0 und 5 einschließlich steht;
• p+m für eine ganze Zahl zwischen 0 und 5 steht;
• R gleich oder verschieden ist und für ein Halogenamtom, einen Hydroxylrest, einen C₁-C₆-Alkylrest, einen C₁-C₆-Monohydroxyalkylrest, einen C₂-C₆-Polyhydroxyalkylrest, einen C₁-C₆-Alkoxyrest, einen Tri-C₁-C₆-alkylsilan-C₁-C₆-alkylrest, einen Amidorest, einen Carboxylrest, einen C₁-C₆-Alkylcarbonylrest, einen Thiorest, einen C₁-C₆-Thioalkylrest, einen C₁-C₆-Alkyl-thiorest, einen Aminorest, einen Aminorest, der durch einen C₁-C₆-Alkyl-, C₁-C₆-Alkylcarbonyl-, Amido- oder C₁-C₆-Alkylsulfonylrest, einen C₁-C₆-Monohydroxyalkylrest oder einen C₂-C₆-Polyhydroxyalkylrest substituiert ist, steht; mit der Maßgabe, daß die Reste R an einem Kohlenstoffatom stehen;
• R₃ gleich oder verschieden ist und für einen C₁-C₆-Alkylrest, einen C₁-C₆-Monohydroxyalkylrest, einen C₂-C₆-Polyhydroxyalkylrest, einen Tri-C₁-C₆-alkylsilan-C₁-C₆-alkylrest, einen C₁-C₆-Alkoxy-C₁-C₆-alkylrest, einen C₁-C₆-Carbamyl-alkylrest, einen C₁-C₆-Alkylcarboxy-C₁₋₆-alkylrest oder einen Benzylrest steht; mit der Maßgabe, daß die Reste R₃ an einem Stickstoffatom stehen;
• R₇ für einen C₁-C₆-Alkylrest; einen C₁-C₆-Mono-hydroxyalkylrest; einen C₂-C₆-Polyhydroxyalkyl-rest; einen Arylrest; einen Benzylrest; einen C₁-C₆-Aminoalkylrest; einen C₁-C₆-Aminoalkylrest, dessen Amin ein- oder zweifach durch einen C₁-C₆-Alkyl-, C₁-C₆-Alkylcarbonyl-, Amido- oder C₁-C₆-Alkylsulfonylrest substituiert ist; einen C₁-C₆-Carboxyalkylrest; einen C₁-C₆-Carbamylalkyl-rest; einen C₁-C₆-Trifluoralkylrest; einen Tri-C₁-C₆-alkylsilan-C₁-C₆-alkylrest; einen C₁-C₆-Sulfonamidoalkylrest; einen C₁-C₆-Alkylcarboxy-C₁-C₆-alkylrest; einen C₁-C₆-Alkylsulfinyl-C₁-C₆-alkylrest; einen C₁-C₆-Alkylsulfonyl-C₁-C₆-alkylrest; einen C₁-C₆-Alkylcarxy-C₁-C₆-alkylrest, einen N-C₁-C₆-Alkylcarbamyl-C₁-C₆-alkylrest oder einen N-C₁-C₆-Alkylsulfonamido-C₁-C₆-alkylrest steht;
• x für 0 oder 1 steht,
- im Fall von x = 0 der Linkerarm D an das Stickstoffatom gebunden ist,
- im Fall von x = 1 der Linkerarm D an eines der Ringglieder E, G, J, L oder M gebunden ist, Y⁻ für ein Gegenion steht.

2. Derivate nach Anspruch 1, worin n gleich 0 ist.

3. Derivate nach Anspruch 1, worin n gleich 1 ist und R₁ für ein Halogenatom oder eine gesättigte oder ungesättigte, aliphatische oder alicyclische C₁-C₆-Kohlenwasserstoffkette, wobei ein oder mehrere Kohlenstoffatome durch ein Sauerstoff-, Stickstoff-, Silicium- oder Schwefelatom oder eine SO₂-Gruppe ersetzt sein können, steht, wobei der Rest R₁ weder eine Peroxidbindung noch Diazo-, Nitro- oder Nitrosoreste enthält.

4. Derivate nach Anspruch 1, worin R₁ unter Chlor, Brom, einem C₁-C₄-Alkylrest, einem C₁-C₄-Hydroxyalkylrest, einem C₁-C₄-Aminoalkylrest, einem C₁-C₄-Alkoxyrest oder einem C₁-C₄-Hydroxyalkoxyrest ausgewählt ist.

5. Derivate nach Anspruch 4, worin R₁ unter einem Methylrest, einem Hydroxymethylrest, einem 2-Hydroxyethylrest, einem 1,2-Dihydroxyethylrest, einem Methoxyrest, einem Isopropyloxyrest oder einem 2-Hydroxyethoxyrest ausgewählt ist.

6. Derivate nach einem der Ansprüche 1 bis 5, worin R₂ der Formel (II) so beschaffen ist, daß x gleich 0 ist und R₅ und R₆ separat unter einem C₁-C₆-Alkylrest, einem C₁-C₄-Monohydroxyalkylrest, einem C₂-C₄-Polyhydroxyalkylrest, einem C₁-C₆-Alkoxy-C₁-C₄-alkylrest, einem C₁-C₆-Amidoalkylrest oder einem Tri-C₁-C₆-alkylsilan-C₁-C₆-alkylrest ausgewählt sind, oder R₄ mit R₅ zusammen einen Azetidin-, Pyrrolidin-, Piperidin-, Piperazin- oder Morpholinring bildet, wobei R₆ in diesem Fall unter einem C₁-C₆-Alkylrest; einem C₁-C₆-Monohydroxyalkyl-rest; einem C₂-C₆-Polyhydroxyalkylrest; einem C₁-C₆-Aminoalkylrest; einem Aminoalkylrest, der ein-oder zweifach durch einen C₁-C₆-Alkyl-, C₁-C₆-Alkylcarbonyl-, Amido- oder C₁-C₆-Alkylsulfonylrest substituiert ist; einem C₁-C₆-Carbamylalkylrest; einem Tri-C₁-C₆-alkylsilan-C₁-C₆-alkylrest; einem C₁-C₆-Alkylcarboxy-C₁-C₆-alkylrest; einem C₁-C₆-Alkylcarbonyl-C₁-C₆-alkylrest oder einem N-C₁-C₆-Alkylcarbamyl-C₁-C₆-alkylrest ausgewählt ist.

7. Derivate nach einem der Ansprüche 1 bis 5, worin R₂ der Formel (II) so beschaffen ist, daß x gleich 1 ist, R₇ unter einem C₁-C₆-Alkylrest; einem C₁-C₆-Monohydroxyalkylrest; einem C₂-C₆-Polyhydroxyalkyl-rest; einem C₁-C₆-Aminoalkylrest; einem C₁-C₆-Aminoalkylrest, dessen Amin ein- oder zweifach durch einen C₁-C₆-Alkyl-, C₁-C₆-Alkylcarbonyl-, Amido- oder C₁-C₆-Alkylsulfonylrest substituiert ist; einem C₁-C₆-Carbamylalkylrest; einem Tri-C₁-C₆-alkylsilan-C₁-C₆-alkylrest; einem C₁-C₆-Alkylcarboxy-C₁-C₆-alkylrest; einem C₁-C₆-Alkylcarbonyl-C₁-C₆-alkylrest oder einem N-C₁-C₆-Alkylcarbamyl-C₁-C₆-alkylrest ausgewählt ist; R₄ mit R₅ zusammen einen Azetidin-, Pyrrolidin-, Piperidin-, Piperazin- oder Morpholinring bildet, wobei R₆ in diesem Fall unter einem C₁-C₆-Alkylrest; einem C₁-C₆-Monohydroxyalkylrest; einem C₂-C₆-Polyhydroxyalkylrest; einem C₁-C₆-Aminoalkylrest; einem C₁-C₆-Aminoalkylrest, dessen Amin ein- oder zweifach durch einen C₁-C₆-Alkyl-, C₁-C₆-Alkylcarbonyl-, Amido- oder C₁-C₆-Alkylsulfonylrest substituiert ist; einem C₁-C₆-Carbamylalkylrest; einem Tri-C₁-C₆-alkylsilan-C₁-C₆-alkylrest; einem C₁-C₆-Alkylcarboxy-C₁-C₆-alkylrest; einem C₁-C₆-Alkylcarbonyl-C₁-C₆-alkylrest oder einem N-C₁-C₆-Alkylcarbamyl-C₁-C₆-alkylrest ausgewählt ist.

8. Derivate nach einem der Ansprüche 1 bis 7, worin D für eine kovalente Bindung oder eine gegebenenfalls substituierte Alkylenkette steht.

9. Derivate nach Anspruch 8, worin R₂ der Formel (III) so beschaffen ist, daß die Ringglieder E, G, J und L einen Pyrrol-, Imidazol-, Pyrazol-, Oxazol-, Thiazol- oder Triazolring bilden.

10. Derivate nach Anspruch 9, worin die Ringglieder E, G, J und L einen Imidazolring bilden.

11. Derivate nach Anspruch 1, worin R₂ der Formel (IV) so beschaffen ist, daß die Ringglieder E, G, J, L und M mit dem Stickstoff des Rings einen unter den Pyridin- und Pyrimidinringen ausgewählten Ring bilden.

12. Derivate nach einem der Ansprüche 1 bis 11, worin R₂ der Formel (III) oder (IV) so beschaffen ist, daß R, R₇ und R₃ für gegebenenfalls substituierte Alkylreste stehen.

13. Derivate nach Anspruch 1, worin R₂ für einen -X-C=NR₈-NR₉R₁₀-Rest steht, X für ein Sauerstoffatom oder einen -NR₁₁-Rest steht, wobei R₈, R₉, R₁₀ und R₁₁ für ein Wasserstoffatom, einen C₁-C₄-Alkylrest oder einen Hydroxyalkylrest stehen.

14. Derivate nach Anspruch 13, worin X für -NR₁₁ steht, R₈ für Wasserstoff steht und R₉ und R₁₀ unter Wasserstoff oder einem Alkylrest ausgewählt sind.

15. Derivate nach einem der Ansprüche 1 bis 14, ausgewählt unter
- N'-[1-(4-Aminophenyl)pyrrolidin-3-yl]-N,N-di-methylguanidin
- N-[1-(4-Aminophenyl)pyrrolidin-3-yl]guanidin
- 3-[1-(4-Aminophenyl)pyrrolidin-3-yl]-1-methyl-3H-imidazol-1-iumchlorid
- [1-(4-Aminophenyl)pyrrolidin-3-yl]dimethyl(3-trimethylsilanylpropyl)ammoniumchlorid
- N'-[1-(4-Amino-3-methylphenyl)pyrrolidin-3-yl]-N,N-dimethylguanidin
- N-[1-(4-Amino-3-methylphenyl)pyrrolidin-3-yl]-guanidin
- 3-[1-(4-Amino-3-methylphenyl)pyrrolidin-3-yl]-1-methyl-3H-imidazol-1-iumchlorid
- [1-(4-Amino-3-methylphenyl)pyrrolidin-3-yl]-dimethyl(3-trimethylsilanylpropyl)ammoniumchlorid
- 3-[1-(4-Aminophenyl)pyrrolidin-3-yl]-1-(3-trimethylsilanylpropyl)-3H-imidazol-1-iumchlorid
- 3-[1-(4-Amino-3-methylphenyl)pyrrolidin-3-yl]-1-(3-trimethylsilanylpropyl)-3H-imidazol-1-iumchlorid
- 1'-(4-Aminophenyl)-1-methyl-[1,3']bipyrrolidinyl-1-iumchlorid
- 1'-(4-Amino-3-methylphenyl)-1-methyl-[1,3']bipyrrolidinyl-1-iumchlorid
- 3-{[1-(4-Aminophenyl)pyrrolidin-3-ylcarbamoyl]-methyl}-1-methyl-3H-imidazol-1-iumchlorid
- 3-{[1-(4-Amino-3-methylphenyl)pyrrolidin-3-yl-carbamoyl]methyl}-1-methyl-3H-imidazol-1-iumchlorid.

16. Derivate nach Anspruch 15, ausgewählt unter 1-Methyl-3-[1-(4-aminophenyl)pyrrolidin-3-yl]-3H-imidazol-1-iumchlorid-hydrochlorid, N-[1-(4-Aminophenyl)pyrrolidin-3-yl)guanidin-trihydrochlorid und 1'-(4-Aminophenyl)-1-methyl-[1,3']bipyrrolidinyl-1-iumchlorid.

17. Derivate nach Anspruch 1, worin die Kette D einen Phosphorylrest umfaßt.

18. Derivate nach Anspruch 17, ausgewählt unter [1-(4-Aminophenyl)pyrrolidin-3-yl]oxophosphorylcholin und [1-(4-Amino-3-methylphenyl)pyrrolidin-3-yl]-oxophosphorylcholin.

19. Färbezusammensetzung, umfassend als Oxidationsbase mindestens ein para-Phenylendiaminderivat der Formel (I) gemäß einem der Ansprüche 1 bis 18.

20. Zusammensetzung nach Anspruch 19, ferner umfassend einen Kuppler, der unter meta-Phenylendiaminen, meta-Aminophenolen, meta-Diphenolen, Naphthalin-Kupplern, heterocyclischen Kupplern und Additionssalzen davon ausgewählt ist.

21. Zusammensetzung nach einem der Ansprüche 19 oder 20, umfassend eine zusätzliche, von den Oxidationsbasen der Formel (I) verschiedene Oxidationsbase, die unter para-Phenylendiaminen, Bisphenylalkylendiaminen, para-Aminophenolen, ortho-Aminophenolen, heterocyclischen Basen und Additionssalzen davon ausgewählt ist.

22. Zusammensetzung nach einem der vorhergehenden Ansprüche, in der die Menge jeder der Oxidationsbasen zwischen etwa 0,001 und 10 Gew.-%, bezogen auf das Gesamtgewicht der Färbezusammensetzung, liegt.

23. Zusammensetzung nach Anspruch 22, in der die Menge jeder der Kuppler zwischen etwa 0,001 und 10 Gew.-%, bezogen auf das Gesamtgewicht der Färbezusammensetzung, liegt.

24. Verfahren zum Oxidationsfärben von Keratinfasern, **dadurch gekennzeichnet, daß** man auf die Fasern eine Färbezusammensetzung gemäß einem der Ansprüche 19 bis 23 in Gegenwart eines Oxidationsmittels über einen Zeitraum, der zur Entwicklung der gewünschten Farbe ausreicht, aufbringt.

25. Verfahren nach Anspruch 24, bei dem man das Oxidationsmittel unter Wasserstoffperoxid, Harnstoffperoxid, Alkalimetallbromaten, Persalzen, Persäuren und Oxidaseenzymen auswählt.

26. Vorrichtung mit mehreren Kompartimenten, wobei ein erstes Kompartiment eine Färbezusammensetzung gemäß einem der Ansprüche 19 bis 23 enthält und ein zweites Kompartiment ein Oxidationsmittel enthält.

27. Verwendung der Zusammensetzung gemäß einem der Ansprüche 19 bis 23 zum Färben von Keratinfasern.

28. Nitroverbindungen der folgenden Formel (I') 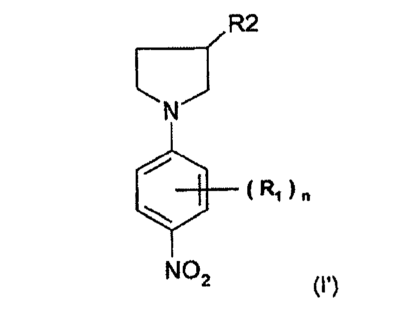 worin R₁, n und R₂ die in einem der Ansprüche 1 bis 21 angegebene Bedeutung besitzen.
